# EUROPEAN PATENT APPLICATION

(11) **EP 4 353 831 A1**
(43) Date of publication of application: **17.04.2024**
(21) Application number: 22819465.0
(22) Date of filing: 06.06.2022
(51) Int. Cl.: C12Q 1/6806, C12Q 1/6876

(54) **PRODUCT AND METHOD FOR ANALYZING OMICS INFORMATION OF SAMPLE**

(30) Priority: 07.06.2021 CN 202110631874
(71) Applicant: Ocean University of China, Qingdao, Shandong 266100 (CN)
(72) Inventor: SHI, Weiyang, Qingdao, Shandong 266100 (CN)
(74) Representative: Weickmann & Weickmann PartmbB
(86) International application number: PCT/CN2022/097049
(87) International publication number: WO 2022/257867

(57) **Abstract**

The present application relates to a product and method for analyzing the omics information of a sample. The method comprises: under the condition of binding a probe to a target molecule, contacting at least one target molecule in a sample with at least one probe, the probe containing a target molecule binding domain capable of specifically binding to the target molecule and at least one reaction blocking modification; carrying out a first processing on a first area of the sample and carrying out a second processing on a second area that is different from the first region in the sample to cause the probe in the first area to be attached to a first tag sequence and the probe in the second area to be attached to a second tag sequence, the second tag sequence being different from the first tag sequence; and determining the composition of the probe attached to the first tag sequence and the composition of the probe attached to the second tag sequence.

## Description

### Field of the Invention

The present application relates to the field of biomedicine, and in particular to a product and method for analyzing the omics information of a sample.

### Background of the Invention

With the gradual completion of the sequencing of the genomes of humans and other species, research in modern biology has entered the post-genomic era. In recent years, high-throughput technologies for the study of different biological macromolecules have been continuously developed, including genomic, transcriptomic, proteomic technologies, etc. Moreover, various omics approaches for small amounts or even single-cell samples have flourished and been applied. With the increasing demand for massive data processing and analysis, bioinformatics has also rapidly developed.

Traditional methods, such as *in situ* hybridization and other multi-detection of different transcripts, have revealed the spatial distribution of gene expression and have aided in elucidating the molecular basis of development and disease. However, these methods cannot simultaneously measure the expression of multiple genes or the presence and/or activity of multiple proteins at various spatial locations in a sample.

Therefore, there is a need for a method capable of simultaneously detecting multiple regions in a sample with high efficiency for analyzing the omics information of multiple regions in the sample.

### Summary of the Invention

The present application provides a method for analyzing the omics information of a sample, which has one or more of the following properties: 1) be capable of performing transcriptomic and proteomic detections with high efficiency; 2) be capable of simultaneously performing multiple detections on omics information.

The present application provides a method, the method includes:
under the condition of specifically binding a probe to a target molecule, contacting at least one target molecule in a sample with at least one probe, the probe containing a target molecule binding domain capable of specifically binding to the target molecule and at least one reaction blocking modification;
carrying out a first processing on a first region of the sample, the first processing being capable of at least partially removing the reaction blocking modification on the probe in the first region, to cause the probe in the first region to be attached to a first tag sequence;
carrying out a second processing on a second region that is different from the first region in the sample, the second processing being capable of at least partially removing the reaction blocking modification on the probe in the second region, to cause the probe in the second region to be attached to a second tag sequence, the second tag sequence being different from the first tag sequence;
determining the composition of the probe attached to the first tag sequence and the probe attached to the second tag sequence, and determining the presence and/or content of the target molecule of the first region and the presence and/or content of the target molecule of the second region in the sample from the composition of the probe.

In some embodiments, the sample includes a tissue section.

In some embodiments, the tissue section has a thickness ranging from 1 to 1000 µm.

In some embodiments, the tissue section in the method is selected from a group consisting of frozen sections, paraffin sections, carbowax sections, ultrathin sections, and plastic sections.

In some embodiments, the tissue section includes a fixed tissue section.

In some embodiments, the tissue section includes a formalin-fixed tissue section.

In some embodiments, the tissue section is a formalin-fixed paraffin section.

In some embodiments, the tissue section is a frozen section.

In some embodiments, the tissue in the tissue section is derived from a multicellular organism.

In some embodiments, the tissue in the tissue section is derived from human.

In some embodiments, the tissue section is a clinically relevant tissue sample.

In some embodiments, the tissue in the tissue section is derived from a group consisting of myocardial tissue, hepatic tissue, splenic tissue, lung tissue, kidney tissue, and brain tissue.

In some embodiments, the first region is a first region on the tissue section, and the second region is a second region that is substantially not overlapped with the first region on the tissue section.

In some embodiments, the sample is a single cell.

In some embodiments, the cell is selected from a group consisting of cells isolated from an organism, cells cultured *in vitro,* primary cells, and discrete cells from an explant.

In some embodiments, the cell in the method is fixed.

In some embodiments, the first region is a first subcellular structure in the cell.

In some embodiments, the second region is a second subcellular structure in the cell.

In some embodiments, the target molecule includes protein, nucleic acid molecules, and/or a lipid.

In some embodiments, the nucleic acid molecule includes a DNA molecule and/or an RNA molecule.

In some embodiments, the DNA includes cDNA.

In some embodiments, the RNA includes cRNA, mRNA, miRNA, IncRNA, and/or eRNA.

In some embodiments, when the target molecule includes RNA, the target molecule binding domain in the probe includes a nucleic acid sequence complementary to the RNA.

In some embodiments, the target molecule binding domain in the probe includes polyA.

In some embodiments, the target molecule binding domain in the probe includes polyT.

In some embodiments, the target molecule binding domain in the probe is at least 50% complementary to the sequence of the target molecule.

In some embodiments, the method includes contacting two or more target molecules in the sample with two or more probes, where the two or more probes are capable of specifically binding the two or more target molecules.

In some embodiments, the two or more target molecules are different.

In some embodiments, in the method, the reaction blocking modification on the probe in the first region is selected from a group consisting of releasable base modified groups, cleavable linkers, and photosensitive groups.

In some embodiments, when the target molecule includes RNA, the reaction blocking modification on the probe in the first region in the method is a cleavable linker.

In some embodiments, the linker in the method is located at 5' end of the probe in the first region.

In some embodiments, the linker in the probe in the first region is cleavable upon the first processing.

In some embodiments, the first processing in the method is selected from a group consisting of electron beam processing, acoustic wave, and illumination.

In some embodiments, the first processing in the method is illumination.

In some embodiments, the light in the method is provided by a light source selected from a group consisting of arc lamp, laser, UV light source, and light emitting diode.

In some embodiments, the light in the method is provided by a UV light source.

In some embodiments, in the method, the reaction blocking modification on the probe in the first region is a releasable base modified group.

In some embodiments, in the method, the reaction blocking modification on the probe in the first region includes at least one releasable base modified group.

In some embodiments, the reaction blocking modification on the probe in the first region includes at least one releasable photosensitive group.

In some embodiments, the base modified group in the probe in the first region includes an NPOM protecting group.

In some embodiments, the base modified group in the probe in the first region is releasable upon the first processing.

In some embodiments, the first processing in the method is selected from a group consisting of electron beam processing, acoustic wave, and illumination.

In some embodiments, the first processing in the method is illumination.

In some embodiments, the light in the method is provided by a light source selected from a group consisting of arc lamp, laser, UV light source, and light emitting diode.

In some embodiments, the light in the method is provided by a UV light source.

In some embodiments, when the target molecule includes RNA, the reaction blocking modification on the probe in the second region is selected from a group consisting of releasable base modified groups, cleavable linkers, and photosensitive groups.

In some embodiments, in the method, the reaction blocking modification on the probe in the second region is a cleavable linker.

In some embodiments, the linker in the method is located at 5' end of the probe in the second region.

In some embodiments, the linker in the probe in the second region is cleavable upon the second processing.

In some embodiments, the second processing in the method is selected from a group consisting of electron beam processing, acoustic wave, and illumination.

In some embodiments, the second processing in the method is illumination.

In some embodiments, the light in the method is provided by a light source selected from a group consisting of arc lamp, laser, UV light source, and light emitting diode.

In some embodiments, the light in the method is provided by a UV light source.

In some embodiments, in the method, the reaction blocking modification on the probe in the second region is a releasable base modified group.

In some embodiments, in the method, the reaction blocking modification on the probe in the second region includes at least one releasable base modified group.

In some embodiments, in the method, the reaction blocking modification on the probe in the second region includes at least one releasable photosensitive group.

In some embodiments, the base modified group in the method includes an NPOM protecting group.

In some embodiments, the base modified group in the probe in the second region is releasable upon the second processing.

In some embodiments, the second processing in the method is selected from a group consisting of electron beam processing, acoustic wave, and illumination.

In some embodiments, the second processing in the method is illumination.

In some embodiments, the light in the method is provided by a light source selected from a group consisting of arc lamp, laser, UV light source, and light emitting diode.

In some embodiments, the light in the method is provided by a UV light source.

In some embodiments, the first processing and the second processing in the method are the same or different.

In some embodiments, during or after carrying out the first processing on the first region of the sample, the second processing is carried out on the second region of the sample.

In some embodiments, the method includes, after the first processing, administering a first tag molecule containing a first tag sequence to the first region of the sample.

In some embodiments, the method includes, after the second processing, administering a second tag molecule containing a second tag sequence to the second region of the sample.

In some embodiments, the probe further includes a first binding domain of an adapter, and the first binding domain of the adapter includes at least one reaction blocking modification.

In some embodiments, in the probe, the first binding domain of the adapter is located at 5' end of the target molecule binding domain.

In some embodiments, the method includes, upon the first processing, the probe in the first region forming a complex with the first tag sequence and an oligonucleotide adapter, and the oligonucleotide adapter includes a probe binding domain and a tag binding domain.

In some embodiments, the first tag sequence in the method includes at least one barcode and a second binding domain of an adapter.

In some embodiments, the method includes, upon the first processing, a nucleic acid sequence in the probe binding domain being complementary to a nucleic acid sequence in the first binding domain of the adapter in the probe in the first region, and a nucleic acid sequence in the tag binding domain being complementary to a nucleic acid sequence in the second binding domain of the adapter in the first tag sequence, thus forming a partially double-stranded structure.

In some embodiments, the second binding domain of the adapter in the first tag sequence is linked to the first binding domain of the adapter in the probe in the first region by a ligase, thus producing the probe attached to the first tag sequence.

In some embodiments, the ligase includes a T4 ligase.

In some embodiments, the barcode of the first tag sequence characterizes spatial location information of the first region.

In some embodiments, the first tag sequence includes a plurality of barcodes.

In some embodiments, the plurality of barcodes in the first tag are different.

In some embodiments, the nucleic acid sequence in the second binding domain of the adapter in the first tag sequence is located at 3' end of the barcode in the first tag sequence.

In some embodiments, the first tag sequence includes a unique molecular identification region.

In some embodiments, the first tag sequence includes a sequencing primer.

In some embodiments, the probe in the second region includes a first binding domain of an adapter, and the first binding domain of the adapter includes at least one reaction blocking modification.

In some embodiments, in the probe, the first binding domain of the adapter is located at 5' end of the target molecule binding domain.

In some embodiments, the method includes, upon the second processing, the probe in the second region forming a complex with the second tag sequence and the oligonucleotide adapter, and the oligonucleotide adapter includes a probe binding domain and a tag binding domain.

In some embodiments, the second tag sequence includes at least one barcode and a second binding domain of an adapter.

In some embodiments, the method includes, upon the second processing, a nucleic acid sequence in the probe binding domain being complementary to a nucleic acid sequence in the first binding domain of the adapter in the probe in the second region, and a nucleic acid sequence in the tag binding domain being complementary to a nucleic acid sequence in the second binding domain of the adapter in the second tag sequence, thus forming a partially double-stranded structure.

In some embodiments, the second binding domain of the adapter in the second tag sequence is linked to the first binding domain of the adapter in the probe in the second region by a ligase, thus producing the probe attached to the second tag sequence.

In some embodiments, the ligase includes a T4 ligase.

In some embodiments, the barcode in the second tag sequence characterizes spatial location information of the second region.

In some embodiments, the second tag sequence includes a plurality of barcodes.

In some embodiments, the plurality of barcodes in the second tag sequence are different.

In some embodiments, the nucleic acid sequence in the second binding domain of the adapter in the second tag sequence is located at 3' end of the barcode in the second tag sequence.

In some embodiments, the second tag sequence includes a unique molecular identification region.

In some embodiments, the second tag sequence includes a sequencing primer.

In some embodiments, at least one target molecule in the sample is subjected to an *in situ* hybridization with at least one probe.

In some embodiments, the method includes, after the *in situ* hybridization, digesting the sample to obtain the probe attached to the first tag sequence and the probe attached to the second tag sequence.

In some embodiments, the method includes amplifying the probe attached to the first tag sequence and the probe attached to the second tag sequence obtained.

In some embodiments, the method further includes, after amplifying the probe attached to the first tag sequence and the probe attached to the second tag sequence obtained, sequencing the probe attached to the first tag sequence and the probe attached to the second tag sequence, to determine the composition of the probe attached to the first tag sequence and the probe attached to the second tag sequence.

In some embodiments, when the target molecule includes protein, the target molecule binding domain in the probe includes a protein binding molecule specifically binding to the protein.

In some embodiments, the protein binding molecule is selected from a group consisting of antibody, peptide, and peptoid.

In some embodiments, when the target molecule includes lipid, the target molecule binding domain in the probe recognizes and binds the target molecule.

In some embodiments, the target molecule includes DNA derived from the sample.

In some embodiments, the DNA includes genomic DNA, open chromatin DNA, a DNA region bound by protein and/or an exogenous nucleic acid linked with protein, lipid and/or small molecule compound, where the protein, lipid and/or small molecule compound is capable of binding the target molecule within the cell.

In some embodiments, the method includes fragmentating the DNA before contacting at least one target molecule in the sample with at least one probe.

In some embodiments, the fragmentation in the method includes integrating a sequence containing the probe into the DNA using a transposase-nucleic acid complex and releasing the transposase.

In some embodiments, the transposase-nucleic acid complex in the method includes a transposase and a transposon terminal nucleic acid molecule, wherein the transposon terminal nucleic acid molecule includes the oligonucleotide adapter sequence.

In some embodiments, the transposase includes Tn5.

In some embodiments, the DNA includes a DNA region bound by protein, and the transposase-nucleic acid complex further includes a portion that directly or indirectly recognizes the protein.

In some embodiments, in the method, the portion that directly or indirectly recognizes the protein includes one or more of a group consisting of an antibody that specifically binds the protein, and protein A or protein G.

In some embodiments, in the method, the reaction blocking modification on the probe in the first region is selected from a group consisting of releasable base modified groups, cleavable linkers, and photosensitive groups.

In some embodiments, the reaction blocking modification on the probe in the first region is a cleavable linker.

In some embodiments, the linker is located at 5' end of the probe in the first region.

In some embodiments, the linker is cleavable upon the first processing.

In some embodiments, the first processing is selected from a group consisting of electron beam processing, acoustic wave, and illumination.

In some embodiments, the first processing is illumination.

In some embodiments, the light is provided by a light source selected from a group consisting of arc lamp, laser, UV light source, and light emitting diode.

In some embodiments, the light is provided by a UV light source.

In some embodiments, the reaction blocking modification on the probe in the first region is a releasable base modified group.

In some embodiments, the reaction blocking modification on the probe in the first region includes at least one releasable base modified group.

In some embodiments, the reaction blocking modification on the probe in the first region includes at least one releasable photosensitive group.

In some embodiments, the base modified group includes an NPOM protecting group.

In some embodiments, the base modified group is releasable upon the first processing.

In some embodiments, the first processing is selected from a group consisting of electron beam processing, acoustic wave, and illumination.

In some embodiments, the first processing is illumination.

In some embodiments, the light is provided by a light source selected from a group consisting of arc lamp, laser, UV light source, and light emitting diode.

In some embodiments, the light is provided by a UV light source.

In some embodiments, the reaction blocking modification on the probe in the second region is selected from a group consisting of releasable base modified groups, cleavable linkers, and photosensitive groups.

In some embodiments, the reaction blocking modification on the probe in the second region is a cleavable linker.

In some embodiments, the linker is located at 5' end of the probe in the second region.

In some embodiments, the linker is cleavable upon the second processing.

In some embodiments, the second processing is selected from a group consisting of electron beam processing, acoustic wave, and illumination.

In some embodiments, the second processing is illumination.

In some embodiments, the light is provided by a light source selected from a group consisting of arc lamp, laser, UV light source, and light emitting diode.

In some embodiments, the light is provided by a UV light source.

In some embodiments, the reaction blocking modification on the probe in the second region is a releasable base modified group.

In some embodiments, the reaction blocking modification on the probe in the second region includes at least one releasable base modified group.

In some embodiments, the base modified group includes an NPOM protecting group.

In some embodiments, the base modified group is releasable upon the second processing.

In some embodiments, the second processing is selected from a group consisting of electron beam processing, acoustic wave, and illumination.

In some embodiments, the second processing is illumination.

In some embodiments, the light is provided by a light source selected from a group consisting of arc lamp, laser, UV light source, and light emitting diode.

In some embodiments, the light is provided by a UV light source.

In some embodiments, the first processing and the second processing are the same or different.

In some embodiments, during or after carrying out the first processing on the first region of the sample, the second processing is carried out on the second region of the sample.

In some embodiments, the method includes, after the first processing, administering the first tag molecule containing the first tag sequence to the first region of the sample.

In some embodiments, the method includes, after the second processing, administering the second tag molecule containing the second tag sequence to the second region of the sample.

In some embodiments, the probe contains a first binding domain of an adapter, and the first binding domain of the adapter includes at least one reaction blocking modification.

In some embodiments, in the probe, the first binding domain of the adapter is located at 5' end of the target molecule binding domain.

In some embodiments, the probe further includes a barcode.

In some embodiments, the barcode in the probe characterizes identity of an antibody.

In some embodiments, the method includes, upon the first processing, the probe in the first region forming a complex with the first tag sequence and an oligonucleotide adapter, and the oligonucleotide adapter includes a probe binding domain and a tag binding domain.

In some embodiments, the first tag sequence includes at least one barcode and a second binding domain of an adapter.

In some embodiments, the method includes, upon the first processing, a nucleic acid sequence in the probe binding domain being complementary to a nucleic acid sequence in the first binding domain of the adapter in the probe in the first region, and a nucleic acid sequence in the tag binding domain being complementary to a nucleic acid sequence in the second binding domain of the adapter in the first tag sequence, thus forming a partially double-stranded structure.

In some embodiments, the second binding domain of the adapter in the first tag sequence is linked to the first binding domain of the adapter in the probe in the first region by a ligase, thus producing the probe attached to the first tag sequence.

In some embodiments, the ligase includes a T4 ligase.

In some embodiments, the barcode of the first tag sequence characterizes spatial location information of the first region.

In some embodiments, the first tag sequence includes a plurality of barcodes.

In some embodiments, the plurality of barcodes in the first tag sequence are different.

In some embodiments, the nucleic acid sequence in the second binding domain of the adapter in the first tag sequence is located at 3' end of the barcode in the first tag sequence.

In some embodiments, the first tag sequence includes a unique molecular identification region.

In some embodiments, the first tag sequence includes a sequencing primer.

In some embodiments, the probe in the second region includes a first binding domain of an adapter, and the first binding domain of the adapter includes at least one reaction blocking modification.

In some embodiments, in the probe, the first binding domain of the adapter is located at 5' end of the target molecule binding domain.

In some embodiments, the method includes, upon the second processing, the probe in the second region forming a complex with the second tag sequence and the oligonucleotide adapter.

In some embodiments, the second tag sequence includes at least one barcode and a second binding domain of an adapter.

In some embodiments, the method includes, upon the second processing, a nucleic acid sequence in the probe binding domain being complementary to a nucleic acid sequence in the first binding domain of the adapter in the probe in the second region, and a nucleic acid sequence in the tag binding domain being complementary to a nucleic acid sequence in the second binding domain of the adapter in the second tag sequence, thus forming a partially double-stranded structure.

In some embodiments, the second binding domain of the adapter in the second tag sequence is linked to the first binding domain of the adapter in the probe in the second region by a ligase, thus producing the probe attached to the second tag sequence.

In some embodiments, the ligase includes a T4 ligase.

In some embodiments, the barcode in the second tag sequence characterizes spatial location information of the second region.

In some embodiments, the second tag sequence includes a plurality of barcodes.

In some embodiments, the plurality of barcodes in the second tag sequence are different.

In some embodiments, the nucleic acid sequence in the second binding domain of the adapter in the second tag sequence is located at 3' end of the barcode in the second tag sequence.

In some embodiments, the second tag sequence includes a unique molecular identification region.

In some embodiments, the second tag sequence includes a sequencing primer.

In some embodiments, the at least one probe specifically recognizes at least one target molecule in the sample.

In some embodiments, the method includes digesting the sample to obtain the probe attached to the first tag sequence and the probe attached to the second tag sequence.

In some embodiments, the method includes amplifying the probe attached to the first tag sequence and the probe attached to the second tag sequence obtained.

In some embodiments, after amplifying the probe attached to the first tag sequence and the probe attached to the second tag sequence obtained, the method further includes sequencing the probe attached to the first tag sequence and the probe attached to the second tag sequence, to determine the composition of the probe attached to the first tag sequence and the probe attached to the second tag sequence.

In another aspect, the present application provides a combination, which includes:
1) a plurality of probe, each of the probes includes a target molecule binding domain, a first binding domain of an adapter and at least one reaction blocking modification;
2) an oligonucleotide adapter, the oligonucleotide adapter includes a probe binding domain and a tag binding domain; and
3) a plurality of tag sequences, each of the tag sequences includes at least one barcode and a second binding domain of an adapter, a nucleic acid sequence in the probe binding domain is complementary to a nucleic acid sequence in the first binding domain of the adapter, and a nucleic acid sequence in the tag binding domain is complementary to a nucleic acid sequence in the second binding domain of the adapter.

In some embodiments, the barcodes in the combination are different in two or more of the plurality of tag sequences.

In some embodiments, the reaction blocking modification in the combination is selected from a group consisting of releasable base modified groups, cleavable linkers, and biotin.

In some embodiments, the reaction blocking modification in the combination is a cleavable linker.

In some embodiments, the reaction blocking modification in the combination is a releasable base modified group.

In some embodiments, in the combination, the 1), 2), and 3) exist independently of each other.

In some embodiments, in the combination, the 1), 2), and 3) exist as a mixture.

In another aspect, the present application provides a kit, which includes the combination.

In some embodiments, the kit includes a transposase.

In some embodiments, the kit further includes at least one of a nucleic acid amplifying agent, a reverse transcriptase, a fixing agent, a permeating agent, a ligating agent, and a lysing agent.

In some embodiments, the kit includes an instruction for use.

In some embodiments, the instruction for use describes the following method:
under the condition of specifically binding a probe to a target molecule, contacting at least one target molecule in a sample with at least one probe, the probe containing a target molecule binding domain capable of specifically binding to the target molecule and at least one reaction blocking modification;
carrying out a first processing on a first region of the sample, the first processing being capable of at least partially removing the reaction blocking modification on the probe in the first region, to cause the probe in the first region to be attached to a first tag sequence in the tag sequence;
carrying out a second processing on a second region that is different from the first region in the sample, the second processing being capable of at least partially removing the reaction blocking modification on the probe in the second region, to cause the probe in the second region to be attached to a second tag sequence in the tag sequence, the second tag sequence being different from the first tag sequence;
determining the composition of the probe attached to the first tag sequence and the probe attached to the second tag sequence, and determining the presence and/or content of the target molecule of the first region and the presence and/or content of the target molecule of the second region in the sample from the composition of the probe.

Other aspects and advantages of the present application can be readily perceived by those skilled in the art from the following detailed description. In the following detailed description, only exemplary embodiments of the present application are shown and described. As will be recognized by those skilled in the art, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are as shown in the appended claims. The characteristics and advantages of the invention involved in the present application can be better understood by referring to the exemplary embodiments described in detail below and the accompanying drawings. A brief description of the drawings is as below:
Fig. 1 shows a schematic diagram of an exemplary method for analyzing the omics information of a sample according to the present application.
Fig. 2 shows a schematic diagram of different regions, e.g., eye and epithelium, of mouse tissue.
Fig. 3 shows the gene expression result of region 1 (eye) of mouse tissue in Fig. 2.
Fig. 4 shows ATAC omics analysis results of region 1 (eye) and region 2 (epithelium) of mouse tissue in Fig. 2

### Detailed Description of the Embodiments

The implementation of the present invention is described below with reference to specific embodiments, and other advantages and effects of the present invention will be readily known to those familiar with the art from the contents disclosed in the present specification.

### Definition of terms

In the present application, the term "characterize" generally refers to a description of the information on a nucleic acid and other relevant molecules obtained by sequencing or other biological analyzing methods such as genomics and/or proteomics. For example, the information may include sequence information from whole genome sequencing, information on accessible chromatin sequences and distribution, information on the binding of nucleic acid sequences to their binding factors, information on mutations in disease-causing genes, single nucleotide polymorphism (SNP), nucleotide methylation, transcriptomic information (e.g., temporal or spatial variations in gene expression levels), etc.

In the present application, the term "sequencing" generally refers to a technology for obtaining the information of a nucleic acid molecule sequence. For example, for analyzing the base sequences of specific DNA fragments (e.g., arrangements of adenine (A), thymine (T), cytosine (C), and guanine (G), etc.); the sequencing method may include Sanger Dideoxy Chain Termination Method, Pyrosequencing, and "parallel sequencing by synthesis" or "sequencing by ligation" platforms used by Illumina, Life Technologies and Roche for new-generation sequencing, sequencer from MGI Tech/Complete Genomics; generally, the sequencing method may also include nanopore sequencing method, e.g., the method developed by Oxford Nanopore Technologies, the third-generation sequencer from PacBio, or an electronic detection-based method, e.g., Ion Torrent technology launched by Life Technologies, etc.

In the present application, the term "protein A" generally refers to a protein derived from cells that can bind to the conserved regions of heavy chains of antibodies from different species, i.e., the recognition proteins of antibodies. For example, protein A can bind to the Fc fragment of IgG molecules from the sera of human and various mammals, where the mammals may include pigs, dogs, rabbits, human, monkeys, rats, mice, and cows, etc.; the subclasses of IgG to which protein A binds may mainly include IgG1, IgG2, and IgG4; in addition to binding to IgG, protein A can also bind to IgM and IgA in the sera. For example, protein A may include Staphylococcal Protein A (SPA). SPA is a major component of the cell wall antigen and present in almost 90% or more of *Staphylococcus aureus* strains. However, there is a significant variation in the content of this component among different strains. The functionality of Protein A to bind to antibodies allows for the localization and/or analysis of target proteins through the formation of target protein-antibody-Protein A complexes.

In the present application, the term "unique molecular identification region" may also be referred to as "molecular barcode", "molecular marker", "Unique identifier (UID)", "Unique molecular identifier (UMI)", etc., and generally refers to encoding a unique sequence on each original nucleotide fragment in the same sample. It can typically be designed as a completely random nucleotide chain (e.g., NNNNNNN), a partially degenerate nucleotide chain (e.g., NNNRNYN), or a designated nucleotide chain (e.g., when template molecules are limited). The design, incorporation, and application of UMI may be carried out in a manner known in the art, for example as disclosed in WO 2012/142213 to Islam et, al. (Nat. Methods) (2014) 11:163-166, and (Nat. Methods) (2012) 9: 72-74 to Kivioja, T. et, al., which are incorporated herein by reference in their entirety.

In the present application, the term "barcode" generally refers to a nucleotide sequence or a derived or modified form thereof that can identify the source of the probe. For example, the barcode can identify probes derived from different regions.

In the present application, the term "oligonucleotide adapter" generally refers to a nucleotide sequence that can be complementary to the first binding domain of the adapter in the probe and can be complementary to the second binding domain of the adapter in the tag sequence. The nucleotide sequence may be a partially double-stranded structure, for example, it may have a prominent sequence that hybridizes with the tag sequence. In some embodiments, the oligonucleotide adapter may also include an amplification primer recognition sequence. In some embodiments, the oligonucleotide adapter may also include a reverse transcription primer sequence.

In the present application, the term "transposase-nucleic acid complex" generally refers to a complex formed from a transposase and a sequence containing the first binding domain of the adapter in the probe. The transposase generally refers to an enzyme capable of binding to the transposon ends and catalyzing its movement to other parts of the genome through mechanisms such as cut-and-paste or replicative transposition. The transposon generally refers to a segment of nucleotides that can move freely within the genome, a concept first proposed by Barbara McClintock in the late 1940s during her studies on the genetic mechanisms of corn. Subsequent research by other groups elucidated the molecular basis of transposition. For example, McClintock discovered that chromosomal segments could change positions, jumping from one chromosome to another. The repositioning of these transposons can alter the expression of other genes. For instance, in maize, transposons can cause changes in color. In other organisms such as bacteria, they can induce antibiotic resistance during the process of human evolution. The transposase-nucleic acid complex can include a dimer formed by two transposases, each binding to the first binding domain of the adapter in the probe, where the two transposases may be either the same or different.

In the present application, the term "Tn5" generally refers to a Tn5 transposase, a member of ribonuclease (RNase) superfamily. Tn5 can be found in *Shewanella oneidensis* and *Escherichia coli.* Tn5 may include naturally occurring Tn5 transposases and various active mutated forms thereof. Similar to most of other transposases, Tn5 contains a DDE motif, which serves as the catalytic active site for mediating the transposition of transposons. It has been reported that the DDE motif can coordinate with divalent metal ions, such as magnesium and manganese, playing a crucial role in catalyzing the reaction. The transposase Tn5 may have elevated transposition activity and catalyze the movement of transposons through mutations in the DDE region. For example, glutamic acid at position 326 is converted to aspartic acid, and two aspartic acids at positions 97 and 188 are converted to glutamic acid (based on the amino acid numbering of the amino acid sequence of GenBank accession number YP_001446289).

In the present application, the term "hybridized," "hybridizable," or "complementary" generally mean that a nucleic acid (e.g., RNA, DNA) includes a nucleotide sequence capable of specifically binding to another nucleic acid sequence non-covalently (i.e., forming a Watson-Crick base pair and/or a G/U base pair) under *in vitro* and/or *in vivo* conditions of appropriate temperature and solution ionic strength. The Watson-Crick base pairing includes adenine/adenosine (A) paired with thymidine/thymine (T), A paired with uracil/uridine (U), guanine/guanosine (G) paired with cytosine/cytidine (C). In some embodiments, G may also be paired with a U base for hybridization between two RNA molecules (e.g., dsRNA), or for hybridization between DNA molecules and RNA molecules (e.g., when the bases of a DNA target nucleic acid are paired with a guide RNA, etc.). Hybridization requires that the two nucleic acids contain complementary sequences, but the possible mismatch between bases cannot be ruled out. The conditions suitable for hybridization between two nucleic acids depend on the length and degree of complementarity of the nucleic acids, which are well known in the art. The greater the degree of complementarity between two nucleotide sequences, the greater the value of the melting temperature (Tm) of the hybrids of nucleic acids having these complementary sequences.

In the present application, the "releasable base modified groups" generally refers to base modified groups that can be released from the probe after processing. For example, the "releasable base modified groups" may include photosensitive groups. In some instances, by introducing photosensitive groups, the reaction (e.g., complementary hybridization) can be controlled. For example, photosensitive groups may include photocleavable groups and photoisomerization groups. Upon exposure to light, the photosensitive groups can absorb energy, undergo electron transfer and energy-level transition, resulting in the cleavage of the bond and the release of the modified probe. For example, photosensitive groups may include o-nitrobenzyl (NPP) and derivatives thereof. For example, photosensitive groups may include 4,5-dimethoxy-2-nitrobenzyl (DMNB) and/or 6-nitro-3,4-methylenedioxybenzoxymethyl (NPOM).

In the present application, the "cleavable linkers" generally refers to a segment of nucleotide sequence that is cleavable upon processing (e.g., optical processing). In some embodiments, the nucleotide may be a segment of fixed nucleotide sequence. For example, the cleavable linkers may include photocleavable linkers (PC linkers). For example, the photocleavable linkers can be located between DNA bases. For example, the 5'-phosphate group of the probe can be exposed after the photocleavable linker is broken.

In the present application, the "substantially not overlapped" generally means that the overlapped portion between the first region and the second region approaches 0. For example, the first region is not overlapped with the second region.

### Detailed description of the invention

In one aspect, the present application provides a method, which includes:
under the condition of specifically binding a probe to a target molecule, contacting at least one target molecule in a sample with at least one probe, the probe containing a target molecule binding domain capable of specifically binding to the target molecule and at least one reaction blocking modification;
carrying out a first processing on a first region of the sample, the first processing being capable of at least partially removing the reaction blocking modification on the probe in the first region, to cause the probe in the first region to be attached to a first tag sequence;
carrying out a second processing on a second region that is different from the first region in the sample, the second processing being capable of at least partially removing the reaction blocking modification on the probe in the second region, to cause the probe in the second region to be attached to a second tag sequence, the second tag sequence being different from the first tag sequence;
determining the composition of the probe attached to the first tag sequence and the probe attached to the second tag sequence, and determining the presence and/or content of the target molecule of the first region and the presence and/or content of the target molecule of the second region in the sample from the composition of the probe.

In another aspect, the present application further provides a method for analyzing the omics information of a sample, which includes:
under the condition of specifically binding a probe to a target molecule, contacting at least one target molecule in a sample with at least one probe, the probe containing a target molecule binding domain capable of specifically binding to the target molecule and at least one reaction blocking modification;
carrying out a first processing on a first region of the sample, the first processing being capable of at least partially removing the reaction blocking modification on the probe in the first region, to cause the probe in the first region to be attached to a first tag sequence;
carrying out a second processing on a second region that is different from the first region in the sample, the second processing being capable of at least partially removing the reaction blocking modification on the probe in the second region, to cause the probe in the second region to be attached to a second tag sequence, the second tag sequence being different from the first tag sequence;
determining the composition of the probe attached to the first tag sequence and the probe attached to the second tag sequence, and determining the presence and/or content of the target molecule of the first region and the presence and/or content of the target molecule of the second region in the sample from the composition of the probe.

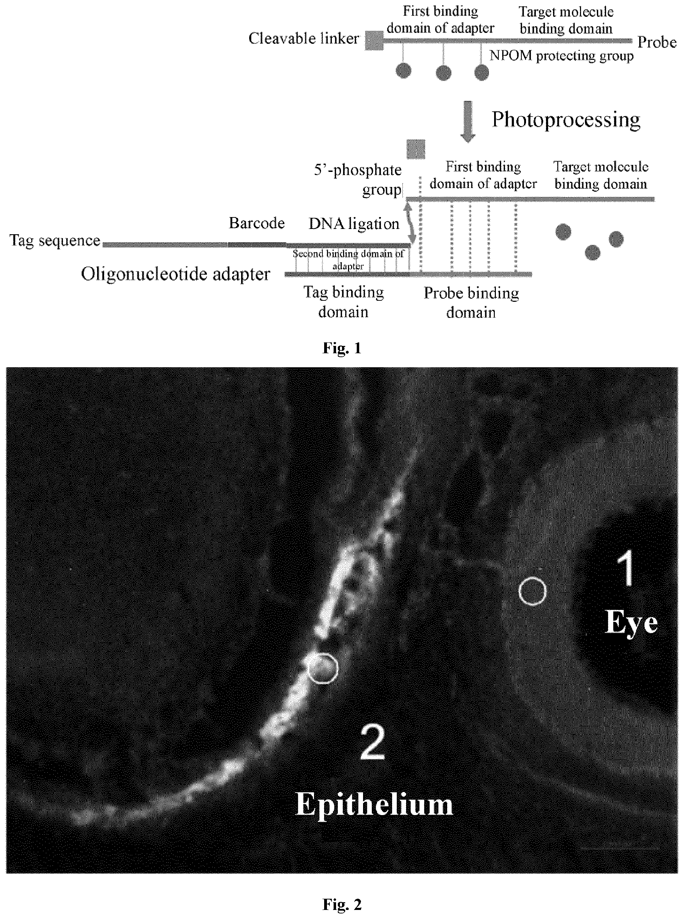

A schematic diagram for an exemplary method for analyzing the omics information of a sample is shown in Fig. 1.

In another aspect, the present application provides a sequencing method, which includes:
under the condition of specifically binding a probe to a target molecule, contacting at least one target molecule in a sample with at least one probe, the probe containing a target molecule binding domain capable of specifically binding to the target molecule and at least one reaction blocking modification;
carrying out a first processing on a first region of the sample, the first processing being capable of at least partially removing the reaction blocking modification on the probe in the first region, to cause the probe in the first region to be attached to a first tag sequence;
carrying out a second processing on a second region that is different from the first region in the sample, the second processing being capable of at least partially removing the reaction blocking modification on the probe in the second region, to cause the probe in the second region to be attached to a second tag sequence, the second tag sequence being different from the first tag sequence;
sequencing the probe attached to the first tag sequence and the probe attached to the second tag sequence, and determining the presence and/or content of the target molecule of the first region and the presence and/or content of the target molecule of the second region in the sample from the sequencing results.

### Sample

In the present application, the sample may be derived from almost any organism and may include multicellular organisms such as plants, fungi and animals. For example, the sample may be derived from an animal (e.g., a mammal). For example, the sample may be derived from human origin. For example, the sample may be a biopsy tumor or a part thereof. For example, the sample may be a clinically relevant tissue sample. For example, the tumor may include a solid tumor. For example, the tumor may include a hematological tumor.

In the present application, the sample may contain any number of substances, including, but not limited to: cells of almost any organism (including both primary cells and cultured cell lines), cell lysates or extracts (including, but not limited to, RNA extracts, purified mRNA), tissue and tissue extracts (including, but not limited to, RNA extracts, purified mRNA), body fluids (including, but not limited to, blood, urine, serum, lymph, bile, cerebrospinal fluid, interstitial fluid, aqueous or glassy fluid, colostrum, sputum, amniotic fluid, saliva, anal and vaginal secretions, sweat and semen, exudate, exudates (e.g., fluid from abscesses or any other site of infection or inflammation) or fluid from joints (e.g., normal joints or joints affected by diseases such as rheumatoid arthritis, osteoarthritis, gout or suppurative arthritis); the study samples include extracellular fluid, extracellular supernatant from cell culture, inclusion bodies in bacteria, cell compartments, periplasm, and mitochondrial compartments.

In the present application, the sample may include at least one cell. For example, the sample may include one or more, two or more, three or more, four or more, five or more or six or more cells. In the present application, the cells may be selected from a group consisting of cells isolated from an organism, cells cultured *in vitro,* primary cells, and discrete cells from an explant. For example, "cells isolated from an organism" generally refers to cells derived from a multicellular organism. For example, the cells may be isolated from human. For example, "cells cultured *in vitro"* generally refer to cells cultured under suitable culture conditions in an *ex vivo* environment. For example, cells cultured *in vitro* may be cultured in a medium. For example, the "primary cells" generally refer to cells cultured immediately after being taken from an organism. For example, the "discrete cells from an explant" generally refer to discrete cells obtained by hydrolyzing plant tissue.

In the present application, the sample may include a tissue section. In the present application, the tissue section has a thickness ranging from 1 to 1000 µm. For example, the thickness of the tissue section is at least 1 µm, at least 5 µm, at least 20 µm, at least 50 µm, at least 100 µm, at least 150 µm, at least 200 µm, at least 250 µm, at least 300 µm, at least 350 µm, at least 400 µm, at least 450 µm, at least 500 µm, at least 550 µm, at least 600 µm, at least 650 µm, at least 700 µm, at least 750 µm, at least 800 µm, at least 850 µm, at least 900 µm, at least 950 µm.

In the present application, the sample may be selected from a group consisting of frozen sections, paraffin sections, carbowax sections, ultrathin sections, and plastic sections. For example, the tissue section may be a fixed tissue section. For example, the tissue section may be a formalin-fixed tissue section. For example, the tissue section may be a formalin-fixed paraffin section. For example, the tissue section may be a frozen section. For example, the tissue in the tissue section may be derived from a group consisting of myocardial tissue, hepatic tissue, splenic tissue, lung tissue, kidney tissue, and brain tissue.

In the present application, the "frozen section" generally refers to a method of rapidly cooling tissue to a certain hardness at low temperature, followed by sectioning. In the present application, the "paraffin section" generally refers to fixing the tissue and embedding it in paraffin for sectioning. In the present application, the "carbowax section" generally refers to fixing the tissue, washing and then directly embedding it in carbowax for sectioning. In the present application, the "ultrathin section" generally refers to an ultrathin specimen section for transmission electron microscopy, which is typically embedded with resin. In the present application, the "plastic section" generally refers to fixing the tissue and then embedding with plastic for sectioning.

### Probe and target molecule

In the present application, the probe may include a target molecule binding domain. In the present application, the target molecule may include one or more selected from a group consisting of protein, a nucleic acid molecule, a metabolite, and/or lipids. For example, the nucleic acid molecule may include a DNA molecule and/or an RNA molecule. For example, the DNA molecule may be cDNA. For example, the RNA molecule may include cRNA, mRNA, miRNA, IncRNA, and/or eRNA. For example, the RNA may be mRNA. For example, the lipids may include fats, cporesterol, cporesterol esters, phospholipids and/or glycolipids.

In the present application, when the target molecule includes RNA, the target molecule binding domain in the probe includes a nucleic acid sequence complementary to the RNA. In the present application, the target molecule binding domain in the probe may include polyA. For example, the length of the polyA may be at least 1 nucleotide, 2 nucleotides, 3 nucleotides, 4 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides or more. In the present application, the target molecule binding domain in the probe includes polyT. For example, the length of the polyT may be at least 1 nucleotide, 2 nucleotides, 3 nucleotides, 4 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides or more.

In the present application, the target molecule may include mRNA, and the target molecule binding domain of the probe may include a single-stranded nucleic acid. For example, the target molecule hybridizes with the target molecule binding domain. With regard to the hybridization, the conditions suitable for hybridization between two nucleic acids depend on the length and degree of complementarity of the nucleic acids, which are well known in the art. The greater the degree of complementarity between two nucleotide sequences, the greater the value of the melting temperature (Tm) of the hybrids of nucleic acids having these complementary sequences. For example, the single-stranded nucleic acid may include at least 10 nucleotides. For example, the single-stranded nucleic acid may include 10 nucleotides or more, 12 nucleotides or more, 14 nucleotides or more, 16 nucleotides or more, 18 nucleotides or more, 20 nucleotides or more, 22 nucleotides or more, 24 nucleotides or more, 26 nucleotides or more, 28 nucleotides or more, 30 nucleotides or more, 32 nucleotides or more, 34 nucleotides or more, 36 nucleotides or more, 38 nucleotides or more, 40 nucleotides or more, 42 nucleotides or more, 44 nucleotides or more, 46 nucleotides or more, 48 nucleotides or more, 50 nucleotides or more, 52 nucleotides or more, 54 nucleotides or more, 56 nucleotides or more, 58 nucleotides or more or 60 nucleotides or more. For example, the hybridization does not rule out the possibility of mismatches between bases. For example, the target molecule binding domain in the probe is at least 50% complementary to the mRNA 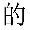 sequence . For example, the complementarity may be 50% or more, 55% or more, 60% or more, 65% or more, 70% or more, 75% or more, 80% or more, 85% or more, 90% or more, 95% or more, 98% or more, 99% or more, 99.5% or more, 99.5% or more. The remaining non-complementary nucleotides may be clustered or scattered with the complementary nucleotides and need not be adjacent to each other or to the complementary nucleotides. For example, polynucleotide may hybridize on one or more segments such that intermediate or adjacent segments are not involved in the hybridization event (e.g., formation of hairpin structures, "bumps", etc.).

In the present application, the target molecule may include protein, and the target molecule binding domain of the probe may include an antibody or an antigen binding fragment capable of specifically binding the target molecule. For example, the antigen binding fragment may include Fab, Fab', F(ab)2, Fv fragment, F(ab')2, scFv, di-scFv, VHH and/or dAb.

In the present application, two or more target molecules in the sample can be contacted with two or more probes. For example, the two or more target molecules may be the same or different.

In the present application, the target molecule may include DNA derived from the sample. For example, the DNA may include genomic DNA, open chromatin DNA, a DNA region bound by protein and/or an exogenous nucleic acid linked with protein, lipid and/or a small molecule compound, the protein, lipid and/or small molecule compound being capable of binding the target molecule within the cell.

In the present application, the method may include fragmentating the DNA before contacting at least one target molecule in the sample with at least one probe. For example, the fragmentation may include integrating a sequence containing the probe into the DNA using a transposase-nucleic acid complex and releasing the transposase. For example, the probe sequence includes a transposon terminal sequence. For example, the transposon terminal sequence is Tn5 or a modified Tn5 transposon terminal sequence. For example, the transposon terminal sequence is a Mu transposon terminal sequence. For example, the Tn5 or modified Tn5 transposon terminal sequence or Mu transposon terminal sequence may include 15 to 25 nucleotides, e.g., 16 nucleotides, 17 nucleotides, 18 nucleotides, 19 nucleotides, 20 nucleotides, 21 nucleotides, 22 nucleotides, 23 nucleotides, 24 nucleotides.

For example, Tn5M and Tn5B can be used as the transposon terminal sequences.

Tn5B: 5'-GTCTCGTGGGCTCGGAGATGTGTATAAGAGACAG-3'; (SEQ ID NO: 11)

Tn5M: 5'-CTGTCTCTTATACACATCT-3'. (SEQ ID NO: 12)

In the present application, DNA can be fragmented using a variety of methods. For example, restriction digestion, utilizing restriction endonucleases, creates cleavage sites in DNA sequences either by blunt-end cutting on both strands or by uneven cutting to generate sticky ends. For example, shear stress-mediated DNA strand disruption may include processes such as ultrasonication, acoustic shear, needle shear, pipetting, or atomizing. Ultrasonication is a type of hydrodynamic shear, exposing DNA sequences to short-term shearing forces, resulting in fragment sizes of approximately 700 bp. Acoustic shear applies high-frequency acoustic energy to DNA samples within a cup-shaped transducer. Needle shear generates shearing forces by passing DNA through a small-diameter needle, physically tearing the DNA into smaller segments. The atomizing force can be generated by passing DNA through small apertures in a nebulizer unit in which the resulting DNA fragments are collected from the fine mist droplets leaving the unit. Typically, these fragments may be of any length between about 200 and about 100000 bases. For example, the fragments would be about 200 bp to about 500 bp, about 500 bp to about 1 kb, about 1 kb to about 10 kb, or about 5 kb to about 50 kb, or about 10 kb to about 30 kb, e.g., about 15 kb to about 25 kb. For example, fragmentation of larger genetic components can be performed by any readily available method, including, for example, commercially available shear-based fragmentation systems (e.g., Covaris fragmentation system), size-targeted fragmentation systems (e.g., Blue Pippin (Sage Sciences)), enzymatic fragmentation methods (e.g., DNA endonucleases, DNA exonucleases), and the like. For example, the fragmentation involves breakage by use of ultrasonication.

For example, the transposase includes Staphylococcus aureus Tn5 (Colegio et, al., J. BacterioL, 183:2384-8, 2001; Kirby C et, al., Mol. Microbiol., 43:173-86, 2002), Tylosin (Tyl) (Devine and Boeke, Nucleic Acids Res., 22:3765-72, 1994, and International Publication WO 95/23875), transposon Tn7 (Craig, N L, Science. 271:1512, 1996; the overview of Craig, N L in Curr Top Microbiol Immunol., 204:27-48, 1996), Tn/O and IS10 (KlecknerN et, al., Curr Top Microbiol Immunol., 204:49-82, 1996), Mariner transposase (Lampe D J et, al., EMBO J., 15:5470-9, 1996), Tel (Plasterk R H, Curr. Topics Microbiol. Immunol., 204: 125-43, 1996), P factors (Gloor, G B, Methods Mol. Biol., 260: 97-114, 2004), Tn3 (Ichikawa and Ohtsubo, J Biol. Chem., 265:18829-32, 1990), bacterial insertion sequences (Ohtsubo and Sekine, Curr. Top. Microbiol. Immunol., 204:1-26, 1996), retroviruses (Brown et, al., Proc Natl Acad Sci USA, 86:2525-9, 1989), and yeast retrotransposon (Boeke and Corces, Annu Rev Microbiol., 43:403-34, 1989), and IS5, Tnl0, Tn903, IS911, and engineered forms of transposase family enzymes (Zhang et, al., (2009), PLoS Genet., 5:el000689. Published electronically on October 16, 2009; Wilson C. et, al. (2007), J. Microbiol. Methods, 71:332-5).

For example, the transposase is a Mu transposase. For example, the transposase is a Tn5 transposase or a Tn10 transposase. The Tn5 transposase is selected from a full-length Tn5 transposase, some functional domains of the Tn5 transposase, mutants of the Tn5 transposase. The Tn10 transposase is selected from a full-length Tn10 transposase, some functional domains of the Tn10 transposase, mutants of the Tn10 transposase. For example, the mutants of Tn5 transposase may be selected from: R30Q, K40Q, Y41H, T47P, E54K/V, M56A, R62Q, D97A, E110K, D188A, Y319A, R322A/K/Q, E326A, K330A/R, K333A, R342A, E344A, E345K, N348A, L372P, S438A, K439A, S445A, G462D, A466D.

For example, the two transposase molecules can bind the same or different double-stranded DNA transposons, such that the insertion sites are marked with 1 or 2 DNA. For example, two transposase molecules (e.g., Tn5 and a hyperactive Tn or another type of transposase containing point mutations) can be assembled with a probe sequence and another standard transposon DNA sequence to form a hybrid transposition complex. Alternatively, only the double-stranded structure 2 described above is used to form a single Tn5 transposition complex. The standard transposon DNA sequence may include an amplification primer sequence and/or a sequencing primer sequence.

For example, the DNA may include a DNA region bound by protein, and the transposase-nucleic acid complex further includes a portion that directly or indirectly recognizes the protein. For example, the portion that directly or indirectly recognizes the protein may include *Staphylococcus aureus* protein A (Protein A), *Streptococcus* protein G (Protein G), *Streptococcus* protein L (Protein L) or other protein analogs with the function of binding antibodies. For example, the portion that directly or indirectly recognizes the protein may also include an antibody that specifically binds the protein. For example, the *Staphylococcus aureus* protein A (Protein A), *Streptococcus* protein G (Protein G), *Streptococcus* protein L (Protein L) or other protein analogs with the function of binding antibodies are each capable of binding the antibody that specifically binds the protein.

For example, the transposase forms fusion protein with the *Staphylococcus aureus* protein A (Protein A), *Streptococcus* protein G (Protein G), *Streptococcus* protein L (Protein L) or other protein analogs with the function of binding antibodies.

For example, the fusion protein forms a complex with the antibody that specifically binds the protein, which then targets the protein.

For example, after the antibody that specifically binds the protein binding to the protein, the fusion protein binds to the antibody to target the protein. For example, the transposition reactions and processes as described herein can be performed in batches.

In the present application, the probe may be provided at a concentration of 100 nM, 70 nM, 60 nM, 50 nM, 40 nM, 30 nM, 20 nM, 10 nM, 9nM, 8 nM, 7nM, 6 nM, 5nM, 4 nM, 3 nM, 2 nM, 1 nM, 0.9 nM, 0.8 nM, 0.7 nM, 0.6 nM, 0.5 nM, 0.4 nM, 0.3 nM, 0.2 nM, 0.1 nM, 0.09 nM, 0.08 nM, 0.07 nM, 0.06 nM, 0.05 nM, 0.04 nM, 0.03 nM, 0.02 nM, 0.01 nM, or less, and any concentration therebetween.

### Reaction blocking modification

In the present application, the reaction blocking modification may be selected from one or more of a group consisting of releasable base modified groups, cleavable linkers, and biotin. For example, the reaction blocking modification can modify 5' end of the probe. For example, the reaction blocking modification can modify 3' end of the probe.

For example, the reaction blocking modification may be a releasable base modified group. For example, the base modified groups may include photosensitive groups. For example, the photosensitive groups may include photocleavable groups and photoisomerization groups. Upon exposure to light, the photosensitive groups can absorb energy, undergo electron transfer and energy-level transition, resulting in the cleavage of the bond and the release of the modified probe. For example, photosensitive groups may include o-nitrobenzyl (NPP) and derivatives thereof. For example, photosensitive groups may include 4,5-dimethoxy-2-nitrobenzyl (DMNB) and/or 6-nitro-3,4-methylenedioxybenzoxymethyl (NPOM). By introducing photosensitive groups, the reaction (e.g., complementary hybridization) can be controlled. In the present application, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20 or more reaction blocking modifications may be included on the probe.

For example, the cleavable linker may be a segment of fixed nucleotide sequence. For example, the cleavable linkers may include photocleavable linkers (PC linkers). For example, the photocleavable linkers can be located between DNA bases. For example, the 5'-phosphate group of the probe can be exposed after the photocleavable linker is broken. In the present application, the sample is placed under excitation light at a wavelength capable of cleaving the linker to photoexcite the sample, thereby cleaving the linker and exposing the 5'-phosphate group.

### Processing

In the present application, two or more regions of the sample can be processed. For example, the two or more regions can be different. For example, at least a first region of the sample can be processed. For example, at least a second region of the sample can be processed. For example, at least a third region, a fourth region, a fifth region, a sixth region, a seventh region, an eighth region, a ninth region, a tenth region, an eleventh region, a twelfth region, a thirteenth region, a fourteenth region, a fifteenth region, a sixteenth region, a seventeenth region, an eighteenth region, a nineteenth region, a twentieth region of the sample can be processed. For example, two or more (e.g., 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more) regions of the sample can be processed at the same time.

In the present application, after the probe specifically binding to the target molecule, a first processing can be carried out on a first region of the sample. For example, the first processing may be selected from a group consisting of electron beam processing, acoustic wave, and illumination. For example, the first processing may be illumination. For example, the light can be provided by a light source selected form a group consisting of arc lamp, laser, UV light source, and light emitting diode. For example, the light can be provided by a UV light source.

In the present application, after the probe specifically binding to the target molecule, a second processing can be carried out on the second region of the sample. For example, the second processing may be selected from a group consisting of electron beam processing, acoustic wave, and illumination. For example, the second processing may be illumination. For example, the light can be provided by a light source selected form a group consisting of arc lamp, laser, UV light source, and light emitting diode. For example, the light can be provided by a UV light source.

### Oligonucleotide adapter

In the present application, the oligonucleotide adapter may include a probe binding domain and a tag binding domain. For example, the probe binding domain in the oligonucleotide adapter may include a single-stranded nucleic acid. For example, the length of the probe binding domain in the oligonucleotide adapter may be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more or 30 nucleotides or more.

In the present application, the probe binding domain of the oligonucleotide adapter may be complementary to the first binding domain of the adapter in the probe. With regard to the probe binding domain, the conditions suitable for hybridization between two nucleic acids depend on the length and degree of complementarity of the nucleic acids, which are well known in the art. The greater the degree of complementarity between two nucleotide sequences, the greater the value of the melting temperature (Tm) of the hybrids of nucleic acids having these complementary sequences. For example, the length of the probe binding domain is sufficient to form a double-stranded structure with the first binding domain of the adapter to which it is complementary.

For example, the length of the tag binding domain in the oligonucleotide adapter may be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more or 30 nucleotides or more.

In the present application, the tag binding domain of the oligonucleotide adapter may be complementary to the second binding domain of the adapter in the tag sequence. With regard to the tag binding domain, the conditions suitable for hybridization between two nucleic acids depend on the length and degree of complementarity of the nucleic acids, which are well known in the art. The greater the degree of complementarity between two nucleotide sequences, the greater the value of the melting temperature (Tm) of the hybrids of nucleic acids having these complementary sequences. For example, the length of the tag binding domain is sufficient to form a double-stranded structure with the second binding domain of the adapter to which it is complementary.

For example, the length of the tag binding domain in the oligonucleotide sequence may be the same as or different from that of the nucleic acid sequence of the probe binding domain.

For example, the double-stranded structure does not rule out the possibility of mismatches between bases. For example, the tag binding domain or the probe binding domain need not be 100% complementary to its complementary sequence. For example, the complementarity may be 60% or more, 65% or more, 70% or more, 75% or more, 80% or more, 85% or more, 90% or more, 95% or more, 98% or more, 99% or more, 99.5% or more. The remaining non-complementary nucleotides may be clustered or scattered with the complementary nucleotides and need not be adjacent to each other or to the complementary nucleotides. For example, polynucleotide may hybridize on one or more segments such that intermediate or adjacent segments are not involved in the hybridization event (e.g., formation of hairpin structures, "bumps", etc.).

### Tag sequence

In the present application, the tag sequence may include at least one barcode and a second binding domain of an adapter. For example, the barcode may characterize spatial location information of different regions. For example, the second binding domain of the adapter in the tag sequence may include a single-stranded nucleic acid. The length of the second binding domain of the adapter may be 1 nucleotide or more, 2 nucleotides or more, 3 nucleotides or more, 5 nucleotides or more, 8 nucleotides or more, 10 nucleotides or more, 12 nucleotides or more, 15 nucleotides or more, 20 nucleotides or more, 22 nucleotides or more, 25 nucleotides or more or 30 nucleotides or more.

In the present application, upon the first processing, the probe in the first region may form a complex with the first tag sequence and an oligonucleotide adapter, and the oligonucleotide adapter includes a probe binding domain and a tag binding domain. For example, upon the first processing, a nucleic acid sequence in the probe binding domain is complementary to a nucleic acid sequence in the first binding domain of the adapter in the probe in the first region, and a nucleic acid sequence in the tag binding domain is complementary to a nucleic acid sequence in the second binding domain of the adapter in the first tag sequence, thus forming a partially double-stranded structure.

For example, the double-stranded structure does not rule out the possibility of mismatches between bases. For example, the tag binding domain or the probe binding domain need not be 100% complementary to its complementary sequence. For example, the complementarity may be 60% or more, 65% or more, 70% or more, 75% or more, 80% or more, 85% or more, 90% or more, 95% or more, 98% or more, 99% or more, 99.5% or more. The remaining non-complementary nucleotides may be clustered or scattered with the complementary nucleotides and need not be adjacent to each other or to the complementary nucleotides. For example, polynucleotide may hybridize on one or more segments such that intermediate or adjacent segments are not involved in the hybridization event (e.g., formation of hairpin structures, "bumps", etc.).

In the present application, the second binding domain of the adapter in the first tag sequence may be linked to the first binding domain of the adapter in the probe in the first region by a ligase, thus producing the probe attached to the first tag sequence.

For example, the ligase may include DNA ligases, such as *Escherichia coli* DNA ligases, T4 DNA ligases, T7 DNA ligases, mammalian ligases (e.g., DNA ligase I, DNA ligase III, DNA ligase IV), thermostable ligases, etc. The T4 DNA ligase may be linked with segments containing DNA, oligonucleotide, RNA, and RNA-DNA hybrids. The ligation may not involve DNA ligases, but employs an alternative such as topoisomerase. Rapid ligation can be achieved by using a high concentration of DNA ligase along with the inclusion of PEG. To select an optimal temperature for the ligation, the preferred temperature of DNA ligase (e.g., it may be 37°C) and the melting temperature of the DNA to be ligated can be considered. The target nucleic acid and barcoded solid support can be suspended in a suitable buffer to minimize the ionization that may impact the ligation. For instance, the ligase may include a T4 ligase.

For example, the barcode of the first tag sequence may characterize spatial location information of the first region. For example, the first tag sequence may include a plurality of barcodes. For example, the plurality of barcodes in the first tag may be different.

In the present application, the nucleic acid sequence in the second binding domain of the adapter in the first tag sequence may be located at 3' end of the barcode in the first tag sequence.

In the present application, the first tag sequence may include a unique molecular identification region. For example, the unique molecular identification region may include a single-stranded nucleic acid. For example, the single-stranded nucleic acid in the unique molecular identification region may include 1 nucleotide or more, 2 nucleotides or more, 3 nucleotides or more, or 4 nucleotides or more, 5 nucleotides or more, 6 nucleotides or more, 7 nucleotides or more or 8 nucleotides or more, 9 nucleotides or more, 12 nucleotides or more, 15 nucleotides or more or 20 nucleotides or more. For example, the single-stranded nucleic acid in the unique molecular identification region may include 8 nucleotides. In the present application, the first tag sequence may include a sequencing primer.

In the present application, upon the second processing, the probe in the second region may form a complex with the second tag sequence and the oligonucleotide adapter.

In the present application, the second tag sequence may include at least one barcode and a first binding domain of an adapter. For example, upon the second processing, a nucleic acid sequence in the probe binding domain is complementary to a nucleic acid sequence in the first binding domain of the adapter in the probe in the second region, and a nucleic acid sequence in the tag binding domain is complementary to a nucleic acid sequence in the second binding domain of the adapter in the second tag sequence, thus forming a partially double-stranded structure.

For example, the double-stranded structure does not rule out the possibility of mismatches between bases. For example, the tag binding domain or the probe binding domain need not be 100% complementary to its complementary sequence. For example, the complementarity may be 60% or more, 65% or more, 70% or more, 75% or more, 80% or more, 85% or more, 90% or more, 95% or more, 98% or more, 99% or more, 99.5% or more. The remaining non-complementary nucleotides may be clustered or scattered with the complementary nucleotides and need not be adjacent to each other or to the complementary nucleotides. For example, polynucleotide may hybridize on one or more segments such that intermediate or adjacent segments are not involved in the hybridization event (e.g., formation of hairpin structures, "bumps", etc.).

For example, the second binding domain of the adapter in the second tag sequence may be linked to the first binding domain of the adapter in the probe in the second region by a ligase, thus producing the probe attached to the second tag sequence.

For example, the ligase may include DNA ligases, such as *Escherichia coli* DNA ligases, T4 DNA ligases, T7 DNA ligases, mammalian ligases (e.g., DNA ligase I, DNA ligase III, DNA ligase IV), thermostable ligases, etc. The T4 DNA ligase may be linked with segments containing DNA, oligonucleotide, RNA, and RNA-DNA hybrids. The ligation may not involve DNA ligases, but employs an alternative such as topoisomerase. Rapid ligation can be achieved by using a high concentration of DNA ligase along with the inclusion of PEG. To select an optimal temperature for the ligation, the preferred temperature of DNA ligase (e.g., it may be 37°C) and the melting temperature of the DNA to be ligated can be considered. The target nucleic acid and barcoded solid support can be suspended in a suitable buffer to minimize the ionization that may impact the ligation. For instance, the ligase may include a T4 ligase.

In the present application, the barcode in the second tag sequence may characterize spatial location information of the second region. For example, the second tag sequence may include a plurality of barcodes. For example, the plurality of barcodes in the second tag sequence may be different.

In the present application, the second tag sequence may include a unique molecular identification region. For example, the unique molecular identification region may include a single-stranded nucleic acid. For example, the single-stranded nucleic acid in the unique molecular identification region may include 1 nucleotide or more, 2 nucleotides or more, 3 nucleotides or more or 4 nucleotides or more, 5 nucleotides or more, 6 nucleotides or more, 7 nucleotides or more or 8 nucleotides or more, 9 nucleotides or more, 12 nucleotides or more, 15 nucleotides or more or 20 nucleotides or more. For example, the single-stranded nucleic acid in the unique molecular identification region may include 8 nucleotides. In the present application, the second tag sequence may include a sequencing primer.

In the present application, two or more regions of the sample can be processed. For example, upon the processing, the probes in the third region, the fourth region, the fifth region, the sixth region, the seventh region, the eighth region, the ninth region, the tenth region, the eleventh region, the twelfth region, the thirteenth region, the fourteenth region, the fifteenth region, the sixteenth region, the seventeenth region, the eighteenth region, the nineteenth region, the twentieth region in the sample may form a complex with the third tag sequence, the fourth tag sequence, the fifth tag sequence, the sixth tag sequence, the seventh tag sequence, the eighth tag sequence, the ninth tag sequence, the tenth tag sequence, the eleventh tag sequence, the twelfth tag sequence, the thirteenth tag sequence, the fourteenth tag sequence, the fifteenth tag sequence, the sixteenth tag sequence, the seventeenth tag sequence, the eighteenth tag sequence, the nineteenth tag sequence, the twentieth tag sequence and the oligonucleotide adapter.

Upon the processing, a nucleic acid sequence in the probe binding domain in the oligonucleotide adapter is complementary to a nucleic acid sequence in the first binding domain of the adapter in the probe in the second region, the third region, the fourth region, the fifth region, the sixth region, the seventh region, the eighth region, the ninth region, the tenth region, the eleventh region, the twelfth region, the thirteenth region, the fourteenth region, the fifteenth region, the sixteenth region, the seventeenth region, the eighteenth region, the nineteenth region, the twentieth region, and a nucleic acid sequence in the tag binding domain is complementary to a nucleic acid sequence in the second binding domain of the adapter in the second tag sequence, the third tag sequence, the fourth tag sequence, the fifth tag sequence, the sixth tag sequence, the seventh tag sequence, the eighth tag sequence, the ninth tag sequence, the tenth tag sequence, the eleventh tag sequence, the twelfth tag sequence, the thirteenth tag sequence, the fourteenth tag sequence, the fifteenth tag sequence, the sixteenth tag sequence, the seventeenth tag sequence, the eighteenth tag sequence, the nineteenth tag sequence, the twentieth tag sequence, thus forming a partially double-stranded structure.

For example, the double-stranded structure does not rule out the possibility of mismatches between bases. For example, the tag binding domain or the probe binding domain need not be 100% complementary to its complementary sequence. For example, the complementarity may be 60% or more, 65% or more, 70% or more, 75% or more, 80% or more, 85% or more, 90% or more, 95% or more, 98% or more, 99% or more, 99.5% or more. The remaining non-complementary nucleotides may be clustered or scattered with the complementary nucleotides and need not be adjacent to each other or to the complementary nucleotides. For example, polynucleotide may hybridize on one or more segments such that intermediate or adjacent segments are not involved in the hybridization event (e.g., formation of hairpin structures, "bumps", etc.).

### Composition and kit

The present application also provides a combination, which includes: a plurality of probes, each of the probes including a target molecule binding domain, a first binding domain of an adapter and at least one reaction blocking modification; an oligonucleotide adapter, the oligonucleotide adapter includes a probe binding domain and a tag binding domain; and a plurality of tag sequences, each of the tag sequences including at least one barcode and a second binding domain of an adapter, a nucleic acid sequence in the probe binding domain is complementary to a nucleic acid sequence in the first binding domain of the adapter, and a nucleic acid sequence in the tag binding domain is complementary to a nucleic acid sequence in the second binding domain of the adapter. For example, the barcodes in two or more of the plurality of tag sequences may be different. For example, the reaction blocking modification may be selected from a group consisting of releasable base modified groups, cleavable linkers, and photosensitive groups. For example, the reaction blocking modification may be a cleavable linker. For example, the reaction blocking modification may be a releasable base modified group. For example, the combination may be a composition.

The present application also provides a kit, which may include the combination of the present application. For example, the kit may further include a transposase. For example, the kit may further include at least one of a nucleic acid amplifying agent, a reverse transcriptase, a fixing agent, a permeating agent, a ligating agent, and a lysing agent. For example, the kit may also include an instruction for use. For example, the instruction for use describes the following method:
under the condition of specifically binding a probe to a target molecule, contacting at least one target molecule in a sample with at least one probe, the probe containing a target molecule binding domain capable of specifically binding to the target molecule and at least one reaction blocking modification;
carrying out a first processing on a first region of the sample, the first processing being capable of at least partially removing the reaction blocking modification on the probe in the first region, to cause the probe in the first region to be attached to a first tag sequence in the tag sequence;
carrying out a second processing on a second region that is different from the first region in the sample, the second processing being capable of at least partially removing the reaction blocking modification on the probe in the second region, to cause the probe in the second region to be attached to a second tag sequence in the tag sequence, the second tag sequence being different from the first tag sequence;
determining the composition of the probe attached to the first tag sequence and the probe attached to the second tag sequence, and determining the presence and/or content of the target molecule of the first region and the presence and/or content of the target molecule of the second region in the sample from the composition of the probe.

Without being limited by any theory, the following examples are only intended to illustrate the composition, the kit, the analyzing method, and the use of the present application and are not intended to limit the scope of the present invention.

### EXAMPLES

### Example 1. Detection of Transcriptome

### 1.1. Design of probe containing reaction blocking modification

### (1) Probe containing cleavable linker (PC Linker)

Probe structure: 5 TC-linker-first binding domain of adapter-UMI-PolyT
Probe sequence:
   5'PC-linker-AGGCCAGAGCATTCG (SEQ ID NO: 1) NNNNNNNNTTTTTTTTTTTTTTTTTTTTTTTTTTTTTT
   where, the sequence of the first binding domain of the adapter is AGGCCAGAGCATTCG (SEQ ID NO: 1), and UMI is NNNNNNNN.

### (2) Design of probe containing NPOM protecting group

Probe sequence:
p-AGGCCAGAGCA (NPOM-T) (NPOM-T) CG (SEQ ID NO: 1) NNNNNNNNTTTTTTTTTTTTTTTTTTTTTTTTTTTTTT

### 1.2 Design of tag sequence and oligonucleotide adapter

(1) Tag sequence
   The tag sequence has a structure of amplification primer recognition region-6bp barcode-second binding domain of adapter
   The specific composition of the tag sequence:
      CCCTACACGACGCTCTTCCGATCT (SEQ ID NO: 3)-6bp barcode-ATCCACGTGCTTGAG (SEQ ID NO: 2)
      where, 6bp barcode is used to distinguish different regions on the section, which can be a combination of any bases where the any bases can be A, T, C and G. The second binding domain of the adapter is ATCCACGTGCTTGAG (SEQ ID NO: 2), and the amplification primer recognition region is CCCTACACGACGCTCTTCCGATCT (SEQ ID NO: 3).
(2) Oligonucleotide adapter
   CGAATGCTCTGGCCTCTCAAGCACGTGGAT (SEQ ID NO: 4)
(3) After annealing, exemplary tag sequence and oligonucleotide adapter can form a partial double-stranded structure I as shown below.

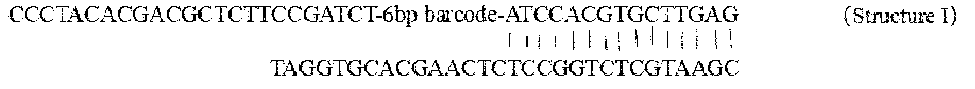
CCCTACACGACGCTCTTCCGATCT-6bp barcode-ATCCACGTGCTTGAG (Structure I)
TAGGTGCACGAACTCTCCGGTCTCGTAAGC

### (1.3) Preparation of Sample

(1) Sample type: Formalin-fixed paraffin section or formaldehyde-fixed frozen section.
(2) The fixed section was subject to antigen exposure processing: First, the sample was processed with an Abcam antigen exposure solution or other buffer at 100°C for 5 min; then digested by a PBS-diluted solution of protease K or pepsin (with a concentration of 1 mg/ml) at 37°C for 10-30min; and finally washed with PBS.

1.4. Reverse transcription reaction was performed with a reaction system comprising 1×reverse transcription buffer, 1 µM dNTP, 2 µM reverse transcription primer, 2 µM reverse transcription primer, 1% Triton, 1% BSA and 2 u/µl reverse transcriptase. The amounts of the above reagents are associated with the sample size. When the tissue section was 1 cm * 1 cm in size, the reaction system was about 50 µl solution.

They were reacted for 5 min at 72°C, and for 10 min at 4°C, and then left at 42°C for 1 hr.

### 1.5 Staining of antibody

(1) One or more antibodies were diluted with 1×PBS buffer containing 1% BSA to a concentration of 1 µg/100 µl.
(2) Primary antibody binding: the antibody solution was reacted with the section at 25°C for 1 hr or at 4°C overnight.
(3) The section was washed with 1×PBS buffer containing 1% BSA.
(4) Secondary antibody binding: the secondary antibody was diluted with 1×PBS buffer containing 1% BSA, and reacted with the section at room temperature for 1 hr.
(5) The section was washed with 1×PBS buffer containing 1% BSA.

1.6 Microscopic Observation: the region of interest in the section was irradiated with 280 nm UV excitation light for 10 s to 5 min.

1.7 The section was washed with 1×PBS buffer containing 1% BSA.

1.8 1×T4 ligase buffer, 1 µM tag sequence, and 1 µl/µl T4 DNA ligase were added to the section, and reacted at room temperature for 15 min. Then, the section was washed with 1×PBS buffer containing 1% BSA to remove the tag sequence.

1.9 The above steps 1.6-1.8 were repeated so that a next region of interest was irradiated, and the different regions of interest on the section were labelled with different tag sequences.

1.10 After completion of all the reactions, the section was digested with a diluted solution of protease K in 1×PBS containing 1% SDS (with a concentration of 20 µg/ml) at 65°C for 1 hr.

1.11 Then, the cDNA was purified with magnetic beads, and a two-strand synthesis was performed in a reaction system comprising the digested cDNA, 1×reverse transcription buffer, 1 µM chain conversion primer, 1 µM dNTP and 1 u/µl reverse transcriptase. The amounts of the above reagents are associated with the sample size. When the tissue section was 1 cm * 1 cm in size, the reaction system was about 50 µl solution.

Where, the chain conversion primer is AAGCAGTGGTATCAACGCAGAGTACATGGG (SEQ ID NO: 14).

After reaction at 42°C for 1 hr, the cDNA was purified with magnetic beads, and then a PCR amplification was performed in a PCR amplification system comprising 1 µM primer 1, 1 µM primer 2, 1×Taq buffer, 1 µM dNTP and 1 u/µl Taq enzyme. The sequence of the primer 1 is AAGCAGTGGTATCAACGCAGAGT (SEQ ID NO: 5), and the sequence of the primer 2 is CCCTACACGACGCTCTTCCGATCT (SEQ ID NO: 3). The amounts of the above reagents are associated with the sample size. When the tissue section was 1 cm * 1 cm in size, the amplification system was about 50 µl solution.

After mixed well, a PCR reaction was performed.
95°C 3 min
95°C 30 s
55°C 30 s
72°C 3 min repeating the above steps for 20-30 cycles
72°C 5 min

1.12 The cDNA was purified, and a library was constructed by an Illumina Nextera kit.
(1) The Illumina Nextera reaction system comprises 1 ng cDNA, 1×Nextera buffer, and 1 µl Nextera enzyme. The reaction volume is 20 µl.
   The reaction ran at 55°C for 7 min.
(2) The reaction product was subject to PCR amplification in a reaction system comprising the reaction product in the (1) above, 1 µM primer 3, 1 µM Illumina N70x primer, 1×Taq buffer, 1 µM dNTP and 1 u/µl Taq enzyme. Where, the primer 3 is CCCTACACGACGCTCTTCCGATCT (SEQ ID NO: 3). The reaction volume is 20 µl.
   After mixed well, a PCR reaction was performed.
   95°C 3 min
   95°C 30 s
   55°C 30 s
   72°C 3 min repeating the above steps for 20-30 cycles.
   72°C 5 min
(3) Sequencing of purified library
(4) According to the barcode in the tag sequence, it is determined which photoprocessed region of interest the final sequencing product comes from.

### Example 2. Detection of Transcriptome Not Based on Reverse Transcription

### 2.1 Design of primer

Primers were designed by a primer design program well known in the art (e.g., primer 3). In the transcriptome gene model of target species, target genes were selected, and 10 pairs of primers were designed for each gene. For adjacent positions in the gene, upstream and downstream primers with length of 25bp were designed, respectively, to ensure their specificity.

### Specific examples:

Upstream primer of each pair of primers: ATCCACGTGCTTGAG (SEQ ID NO: 2)-UMI-probe sequence

Downstream primer of each pair of primers: probe-AGATCGGAAGAGCACACGTCTG (SEQ ID NO: 9)
where, the sequence of UMI is nnnnnnnn (random sequence).

### (1) Synthesis of upstream primer

First, a probe primer set was synthesized in parallel by Twist Bioscience.

The sequence of the probe primer set is:
ATCCACGTGCTTGAG (SEQ ID NO: 2)-nnnnnnnn-probe-GATCACTCTGCGTTGATACCAC (SEQ ID NO: 15)

The library of upstream primers was amplified by PCR with the primers as shown below.
Primer 4: biotin-ATCCACGTGCTTGAG (SEQ ID NO: 2)
Primer 5: GTGGTATCAACGCAGAGTGATC (SEQ ID NO: 6)

Subsequently, the PCR product was purified, and digested by DpnII enzyme to remove the downstream sequence of the probe.

Then, it was bound to streptavidin (briefly, SA) magnetic beads, and the sequence is shown as follows:
Magnetic beads-SA-biotin-ATCCACGTGCTTGAG (SEQ ID NO: 2) nnnnnnnn-probe-

Then, it was denatured by 0.1M NaOH to remove the PCR complementary chain.

Subsequently, it was heated to 100°C to release the forward probe primer bound to the streptavidin magnetic beads.
Biotin-ATCCACGTGCTTGAG (SEQ ID NO: 2) nnnnnnnn-probe-

### (2) Synthesis of downstream primer

A probe primer set was synthesized in parallel by Twist Bioscience.

The sequence of the probe primer set is:
CTTCCGATCTGGTCTCG (SEQ ID NO: 7)-probe-AGATCGGAAGAGCACACGTCTG (SEQ ID NO: 9)

The library of downstream primers was amplified by PCR with the primers as shown below.
Primer 6: biotin-CTTCCGATCTGGTCTCG (SEQ ID NO: 7)
Primer 7: biotin-CAGACGTGTGCTCTTCCGATCT (SEQ ID NO: 8)

The PCR product was bound to the streptavidin magnetic beads, and the excessive primer sequence was removed by digestion with BsaI-HF enzyme.

Subsequently, it was denatured by heating at 95°C to give the final primer as shown below.
Probe-AGATCGGAAGAGCACACGTCTG (SEQ ID NO: 9)

### (3) Preparation of probe containing cleavable linker (PC linker)

Similar to Example 1, a cleavable linker was ligated to the 5' end of the probe.

### (4) Preparation of probe containing NPOM protecting group

Similar to Example 1, an NPOM protecting group was ligated to the 5' end of the probe.

### 2.2 Preparation of Sample, and Hybridization of Probe with Sample

(1) First, the tissue section was fixed with 4% formaldehyde in PBS at room temperature for 10 min, and subsequently the cells were dehydrated and stored in 100% ethanol at -20°C.
(2) Subsequently, a hybridization solution was formulated with components of 10% formamide + 2× sodium citrate buffer (SSC) + 10% dextran sulfate.
(3) At 42°C, the tissue section was pre-hybridized in the hybridization solution for 1 hr.
(4) The upstream primer pool and the downstream primer pool were added into the tissue section with a total concentration of 10 µM.
(5) Then, they were hybridized at 42°C for 12 to 24 hrs.
(6) The tissue section was washed with the hybridization solution twice.
(7) The tissue section was further washed with PBS containing 1% BSA twice.
(8) It was ligated with T4 ligase at room temperature for 1 hr.
(9) The tissue section was further washed with PBS containing 1% BSA twice.

### 2.3 Staining of Antibody

(1) One or more antibodies were diluted with 1×PBS buffer containing 1% BSA to a concentration of 1 µg/100 µl.
(2) Primary antibody binding: the antibody solution was reacted with the section at 25°C for 1 hr or at 4°C overnight.
(3) The section was washed with 1×PBS buffer containing 1% BSA.
(4) Secondary antibody binding: the secondary antibody was diluted with 1×PBS buffer containing 1% BSA, and reacted with the section at room temperature for 1 hr.
(5) The section was washed with 1×PBS buffer containing 1% BSA.

2.4 Microscopic Observation: The region of interest in the section was irradiated with 280 nm UV excitation light for 10 s to 5 min.

2.5 The section was washed with 1×PBS buffer containing 1% BSA.

2.6 1 x T4 ligase buffer, 1 µM tag sequence, and 1 µl/µl T4 DNA ligase were added to the section, and reacted at room temperature for 15 min. Then, the section was washed with 1×PBS buffer containing 1% BSA to remove the tag sequence.

2.7 The above steps 2.4-2.6 were repeated so that a next region of interest was irradiated, and the different regions of interest on the section were labelled with different tag sequences.

2.8 After completion of all the reactions, the section was digested with a diluted solution of protease K in 1 ×PBS containing 1% SDS (with a concentration of 20 µg/ml) at 65°C for 1 hr.

2.9. Library construction and sequencing.

### Example 3 Epigenomic Analysis

3.1. Design of probe containing reaction blocking modification
(1) Probe containing cleavable linker (PC linker)
   Probe for epigenomic analysis:
   5'PC-linker-AGGCCAGAGCATTCGAGATGTGTATAAGAGACAG (SEQ ID NO: 10)
(2) Design of probe containing NPOM protecting group
   p-AGGCCAGAGCA (NPOM-T) (NPOM-T) CGAGATGTGTATAAGAGACAG (SEQ ID NO: 10)

3.2 The above primer and Tn5M primer 5'-P-CTGTCTCTTATACACATCT (SEQ ID NO: 12) were annealed to form a double strand.

### 3.3 Tn5B Primer

Tn5B primer is GTCTCGTGGGCTCGGAGATGTGTATAAGAGACAG (SEQ ID NO: 11), and the Tn5B primer and the Tn5M primer 5'-P-CTGTCTCTTATACACATCT (SEQ ID NO: 12) were annealed to form a double strand.

3.4 Equivalent amounts of the protected Tn5 transposon and the Tn5B transposon were mixed, and further mixed with the Tn5 protein in equimolar amount to assemble a Tn5 enzyme.

3.5 A formaldehyde-fixed frozen section was prepared, and the tissue was permeated with 0.1% Trion 10mM Tris buffer.

3.6 1×TD buffer (available from the Illumina Nextera kit) and 5 µM of the Tn5 enzyme assembled in the above step were added to the section, and subject to ATAC reaction at 37°C for 1 hr.

3.7 The section was washed with 1×PBS buffer containing 1% BSA to remove the unreacted Tn5 enzyme.

3.8 Microscopic observation of the section: the region of interest in the section was irradiated with 280 nm UV exciting light for 10 s to 5 min.

3.9 The section was washed with 1×PBS buffer containing 1% BSA.

3.10 1×T4 ligase buffer, 1 µM tag sequence, and 1 µl/µl T4 DNA ligase were added to the section, and reacted at room temperature for 15 min. Then, the section was washed with 1×PBS buffer containing 1% BSA to remove the tag sequence.

3.11 The above steps 3.8-3.10 were repeated so that a next region of interest was irradiated, and the different regions of interest on the section were labelled with different tag sequences.

3.12 After completion of all the reactions, the section was digested with a diluted solution of protease K in 1 ×PBS containing 1% SDS (with a concentration of 20 µg/ml) at 65°C for 1 hr.

3.13 The DNA was purified with magnetic beads, and then amplified in an amplification system comprising the above reaction product, 1 µM primer 5, 1 µM Illumina N70x primer, 1×Taq buffer, 1 µM dNTP, and 1 u/µl Taq enzyme. The sequence of the primer 8 is CCCTACACGACGCTCTTCCGATCT (SEQ ID NO: 3). The amounts of the above reagents are associated with the sample size. When the tissue section was 1 cm * 1 cm in size, the amplification system was about 50 µl solution.

After mixed well, an amplification was performed.
72c 5 min
95c 3 min
95c 30 s
55c 30 s
72c 3 min repeating the above steps for 20-30 cycles.
72c 5 min

### 3.14 Sequencing of purified library

3.15 According to the barcode in the tag sequence, it is determined which photoprocessed region of interest the final sequencing product comes from.

### Example 4. Multi-Omics Detection of Protein

4.1 The following structure was coupled to the antibody:
Antibody probe: AGGCCAGAGCATTCG (SEQ ID NO: 1) NNNNNNNN-barcode-CTGTCTCTTATACACATCTCCGAGCCCACGAGAC (SEQ ID NO: 13)
where, the 5' end sequence of the antibody probe is the sequence of the first binding domain of the adapter, AGGCCAGAGCATTCG (SEQ ID NO: 1), the 3' end sequence of the antibody probe is CTGTCTCTTATACACATCTCCGAGCCCACGAGAC (SEQ ID NO: 13)

4.2 Similar to Example 1, a cleavable linker or an NPOM protecting group was ligated to the 5' end of the antibody probe. Where, the barcode was used to distinguish different antibodies.

4.3 The 3' end of the antibody probe carries an amino modification, which can be coupled to the target antibody by a common antibody oligo coupling kit.

4.4 Antibody binding: It is the same as the step 2.3 of Example 2.

4.5 When the library was constructed, an additional purification step was added, that is, after digesting the whole tissue section with protease, oligo moieties (less than 100 bp) of the antibody were obtained by purification with magnetic beads and amplified, while moieties with length greater than 100 bp were subject to conventional cDNA or Tn5 amplification.

### Example 5 Analysis of Results

Different regions of mouse tissues were detected. Fig. 2 shows different regions of mouse tissues, such as, eye and epithelium.

The identification results of transcriptome for region 1 (eye) and region 2 (epithelium) of mouse tissues in Fig. 2 are shown in the table below.

| | Number of Reads | Number of Transcripts | Number of Genes |
|---|---|---|---|
| Region 1 | 2072546 | 21461 | 1799 |
| Region 2 | 1723568 | 16132 | 1981 |

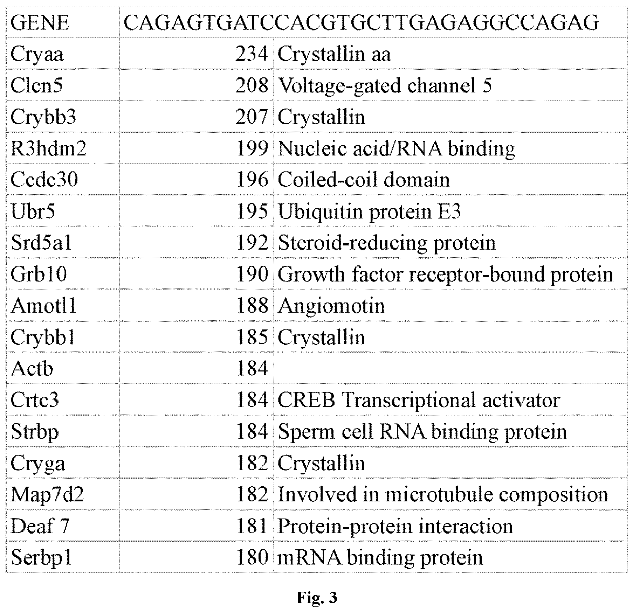

Fig. 3 shows the gene expression result for the region 1 (eye) of the mouse tissues in Fig. 2. The results show that the method of the present application can identify the gene expression specific to eye in the region 1 (eye).

The table below shows the gene expression result for the region 2 (epithelium) of the mouse tissues in Fig. 2. The results show that the method of the present application can identify the gene expression specific to epithelium in the region 2 (epithelium).

| Name of Genes | Number of Transcripts | Function of Genes |
|---|---|---|
| EPCAM | 185 | Epithelial cell adhesion molecule |
| Actin 1 | 142 | Actin |
| LhS28 | 196 | Epithelial ciliary factor |
| beta-Crystallin | 156 | β-crystallin |
| Calcyclin | 134 | Network calcyclin |
| CD151 | 97 | Epithelial cell marker |
| CD166 | 114 | Activated leukocyte adhesion molecule |

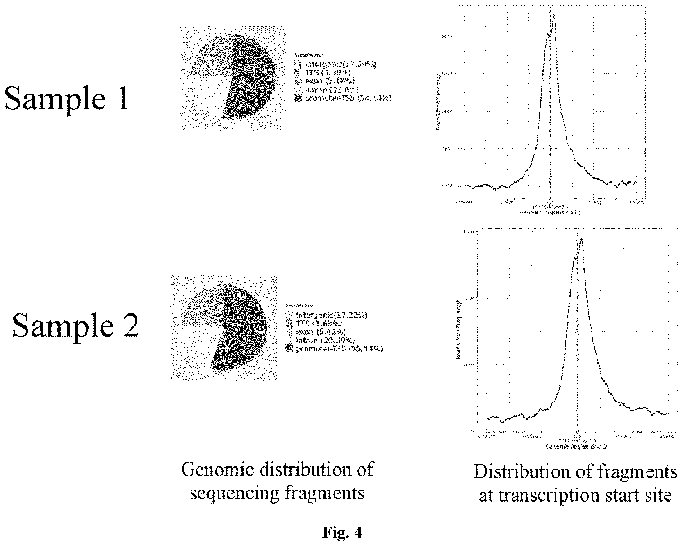

Fig. 4 shows the results of ATAC omics analysis for the region 1 (eyes) and the region 2 (epithelium) of the mouse tissues in Fig. 2. The results show that the genome distribution of the sequenced fragments shows a typical ATAC signal pattern, that is, they were mainly distributed around the transcription start site and exhibited a peak value.

After the tissues in Fig. 2 were fixed, they were stained with an oligo-coupled antibody against actin Actin1, and then subject to a protein quantitative labelling experiment of the present application. The obtained results are shown in the table below.

| | Number of Sequenced Fragments | Number of Unique Molecules |
|---|---|---|
| Region 1 | 175678 | 24 |
| Region 2 | 243569 | 1345 |

It is known that the actin exhibits high expression in the epithelial tissue, but exhibits low expression in the eye, and the number of molecules obtained by the sequencing results are consistent with it.

The above results show that the method of the present application can detect a variety of omics information at the same time.

The foregoing detailed description is provided by way of explanation and examples and is not intended to limit the scope of the appended claims. Various modifications of the embodiments enumerated herein will be apparent to those of ordinary skills in the art and are retained within the scope of the appended claims and equivalent embodiments thereof.

## Claims

1. A method, comprising:
under the condition of specifically binding a probe to a target molecule, contacting at least one target molecule in a sample with at least one probe, the probe containing a target molecule binding domain capable of specifically binding to the target molecule and at least one reaction blocking modification;
carrying out a first processing on a first region of the sample, the first processing being capable of at least partially removing the reaction blocking modification on the probe in the first region, to cause the probe in the first region to be attached to a first tag sequence;
carrying out a second processing on a second region that is different from the first region in the sample, the second processing being capable of at least partially removing the reaction blocking modification on the probe in the second region, to cause the probe in the second region to be attached to a second tag sequence, the second tag sequence being different from the first tag sequence;
determining the composition of the probe attached to the first tag sequence and the probe attached to the second tag sequence, and determining the presence and/or content of the target molecule of the first region and the presence and/or content of the target molecule of the second region in the sample from the composition of the probe.

2. The method according to claim 1, wherein the sample comprises a tissue section.

3. The method according to claim 2, wherein the tissue section has a thickness ranging from 1 to 1000 µm.

4. The method according to any one of claims 2-3, wherein the tissue section is selected from a group consisting of frozen sections, paraffin sections, carbowax sections, ultrathin sections, and plastic sections.

5. The method according to any one of claims 2-4, wherein the tissue section comprises a fixed tissue section.

6. The method according to any one of claims 2-5, wherein the tissue section comprises a formalin-fixed tissue section.

7. The method according to any one of claims 2-6, wherein the tissue section is a formalin-fixed paraffin section.

8. The method according to any one of claims 2-7, wherein the tissue section is a frozen section.

9. The method according to any one of claims 2-8, wherein the tissue in the tissue section is derived from a multicellular organism.

10. The method according to any one of claims 2-9, wherein the tissue in the tissue section is derived from human.

11. The method according to any one of claims 2-10, wherein the tissue section is a clinically relevant tissue sample.

12. The method according to any one of claims 2-11, wherein the tissue in the tissue section is derived from a tissue selected from a group consisting of myocardial tissue, hepatic tissue, splenic tissue, lung tissue, kidney tissue, and brain tissue.

13. The method according to any one of claims 1-12, wherein the first region is a first region on the tissue section, and the second region is a second region that is substantially not overlapped with the first region on the tissue section.

14. The method according to claim 1, wherein the sample is a single cell.

15. The method according to claim 14, wherein the cell is selected from a group consisting of a cell isolated from an organism, a cell cultured *in vitro,* a primary cell, and a discrete cell from an explant.

16. The method according to any one of claims 14-15, wherein the cell is fixed.

17. The method according to any one of claims 14-16, wherein the first region is a first subcellular structure in the cell.

18. The method according to any one of claims 14-17, wherein the second region is a second subcellular structure in the cell.

19. The method according to any one of claims 1-18, wherein the target molecule comprises a protein, a nucleic acid molecule, and/or a lipid.

20. The method according to claim 19, wherein the nucleic acid molecule comprises a DNA molecule and/or an RNA molecule.

21. The method according to claim 20, wherein the DNA comprises cDNA.

22. The method according to any one of claims 20-21, wherein the RNA comprises cRNA, mRNA, miRNA, lncRNA, and/or eRNA.

23. The method according to any one of claims 1-22, wherein, when the target molecule comprises RNA, the target molecule binding domain in the probe comprises a nucleic acid sequence complementary to the RNA.

24. The method according to any one of claims 1-23, wherein the target molecule binding domain in the probe comprises polyA.

25. The method according to any one of claims 1-24, wherein the target molecule binding domain in the probe comprises polyT.

26. The method according to any one of claims 1-25, wherein the target molecule binding domain in the probe is at least 50% complementary to the sequence of the target molecule.

27. The method according to any one of claims 1-26, comprising contacting two or more target molecules in the sample with two or more probes, wherein the two or more probes are capable of specifically binding the two or more target molecules.

28. The method according to 27, wherein the two or more target molecules are different.

29. The method according to any one of claims 20-28, wherein, when the target molecule comprises RNA, the reaction blocking modification on the probe in the first region is selected from a group consisting of a releasable base modified group, a cleavable linker, and a photosensitive group.

30. The method according to claim 29, wherein, when the target molecule comprises RNA, the reaction blocking modification on the probe in the first region is a cleavable linker.

31. The method according to claim 30, wherein the linker is located at 5' end of the probe in the first region.

32. The method according to any one of claims 30-31, wherein the linker is cleavable upon the first processing.

33. The method according to claim 32, wherein the first processing is selected from a group consisting of electron beam processing, acoustic wave, and illumination.

34. The method according to claim 33, wherein the first processing is illumination.

35. The method according to any one of claims 33-34, wherein the light is provided by a light source selected from a group consisting of arc lamp, laser, UV light source, and light emitting diode.

36. The method according to any one of claims 33-35, wherein the light is provided by a UV light source.

37. The method according to any one of claims 29-36, wherein the reaction blocking modification on the probe in the first region is a releasable base modified group.

38. The method according to claim 37, wherein the reaction blocking modification on the probe in the first region comprises at least one releasable base modified group.

39. The method according to any one of claims 37-38, wherein the reaction blocking modification on the probe in the first region comprises at least one releasable photosensitive group.

40. The method according to any one of claims 37-39, wherein the base modified group comprises an NPOM protecting group.

41. The method according to any one of claims 37-40, wherein the base modified group is releasable upon the first processing.

42. The method according to any one of claims 37-41, wherein the first processing is selected from a group consisting of electron beam processing, acoustic wave, and illumination.

43. The method according to claim 42, wherein the first processing is illumination.

44. The method according to any one of claims 42-43, wherein the light is provided by a light source selected from a group consisting of arc lamp, laser, UV light source, and light emitting diode.

45. The method according to any one of claims 42-44, wherein the light is provided by a UV light source.

46. The method according to any one of claims 20-45, wherein when the target molecule comprises RNA, the reaction blocking modification on the probe in the second region is selected from a group consisting of releasable base modified groups, cleavable linkers, and photosensitive groups.

47. The method according to claim 46, wherein the reaction blocking modification on the probe in the second region is a cleavable linker.

48. The method according to claim 47, wherein the linker is located at 5' end of the probe in the second region.

49. The method according to any one of claims 47-48, wherein the linker is cleavable upon the second processing.

50. The method according to claim 49, wherein the second processing is selected from a group consisting of electron beam processing, acoustic wave, and illumination.

51. The method according to any one of claims 49-50, wherein the second processing is illumination.

52. The method according to any one of claims 50-51, wherein the light is provided by a light source selected from a group consisting of arc lamp, laser, UV light source, and light emitting diode.

53. The method according to any one of claims 50-52, wherein the light is provided by a UV light source.

54. The method according to any one of claims 46-53, wherein the reaction blocking modification on the probe in the second region is a releasable base modified group.

55. The method according to claim 54, wherein the reaction blocking modification on the probe in the second region comprises at least one releasable base modified group.

56. The method according to any one of claims 54-55, wherein the reaction blocking modification on the probe in the second region comprises at least one releasable photosensitive group.

57. The method according to any one of claims 54-56, wherein the base modified group comprises an NPOM protecting group.

58. The method according to any one of claims 54-57, wherein the base modified group is releasable upon the second processing.

59. The method according to claim 58, wherein the second processing is selected from a group consisting of electron beam processing, acoustic wave, and illumination.

60. The method according to claim 59, wherein the second processing is illumination.

61. The method according to any one of claims 59-60, wherein the light is provided by a light source selected from a group consisting of arc lamp, laser, UV light source, and light emitting diode.

62. The method according to any one of claims 59-61, wherein the light is provided by a UV light source.

63. The method according to any one of claims 1-62, wherein the first processing and the second processing are the same or different.

64. The method according to any one of claims 1-63, comprising, during or after carrying out the first processing on the first region of the sample, carrying out the second processing on the second region of the sample.

65. The method according to any one of claims 1-64, comprising, after the first processing, administering a first tag molecule containing a first tag sequence to the first region of the sample.

66. The method according to any one of claims 1-65, comprising, after the second processing, administering a second tag molecule containing a second tag sequence to the second region of the sample.

67. The method according to any one of claims 1-66, wherein the probe in the first region comprises a first binding domain of an adapter, and the first binding domain of the adapter comprises at least one reaction blocking modification.

68. The method according to claim 67, in the probe, the first binding domain of the adapter is located at 5' end of the target molecule binding domain.

69. The method according to any one of claims 1-68, further comprising, upon the first processing, the probe in the first region forming a complex with the first tag sequence and an oligonucleotide adapter, and the oligonucleotide adapter comprises a probe binding domain and a tag binding domain.

70. The method according to any one of claims 1-69, wherein the first tag sequence comprises at least one barcode and a second binding domain of an adapter.

71. The method according to any one of claims 69-70, further comprising, upon the first processing, a nucleic acid sequence in the probe binding domain being complementary to a nucleic acid sequence in the first binding domain of the adapter in the probe in the first region, and a nucleic acid sequence in the tag binding domain being complementary to a nucleic acid sequence in the second binding domain of the adapter in the first tag sequence, thus forming a partially double-stranded structure.

72. The method according to any one of claims 70-71, wherein the second binding domain of the adapter in the first tag sequence is linked to the first binding domain of the adapter in the probe in the first region by a ligase, thus producing the probe attached to the first tag sequence.

73. The method according to claim 72, wherein the ligase comprises a T4 ligase.

74. The method according to any one of claims 70-73, wherein the barcode in the first tag sequence characterizes spatial location information of the first region.

75. The method according to any one of claims 70-74, wherein the first tag sequence comprises a plurality of barcodes.

76. The method according to claim 75, wherein the plurality of barcodes in the first tag sequence are different.

77. The method according to any one of claims 70-76, wherein the nucleic acid sequence in the second binding domain of the adapter in the first tag sequence is located at 3' end of the barcode in the first tag sequence.

78. The method according to any one of claims 1-77, wherein the first tag sequence comprises a unique molecular identification region.

79. The method according to any one of claims 1-78, wherein the first tag sequence comprises a sequencing primer.

80. The method according to any one of claims 1-79, wherein the probe in the second region comprises a first binding domain of an adapter, and the first binding domain of the adapter comprises at least one reaction blocking modification.

81. The method according to claim 80, in the probe, the first binding domain of the adapter is located at 5' end of the target molecule binding domain.

82. The method according to any one of claims 1-81, further comprising, upon the second processing, the probe in the second region forming a complex with the second tag sequence and the oligonucleotide adapter, and the oligonucleotide adapter comprises a probe binding domain and a tag binding domain.

83. The method according to any one of claims 1-82, wherein the second tag sequence comprises at least one barcode and a second binding domain of an adapter.

84. The method according to any one of claims 82-83, further comprising, upon the second processing, a nucleic acid sequence in the probe binding domain being complementary to a nucleic acid sequence in the first binding domain of the adapter in the probe in the second region, and a nucleic acid sequence in the tag binding domain being complementary to a nucleic acid sequence in the second binding domain of the adapter in the second tag sequence, thus forming a partially double-stranded structure.

85. The method according to any one of claims 83-84, wherein the second binding domain of the adapter in the second tag sequence is linked to the first binding domain of the adapter in the probe in the second region by a ligase, thus producing the probe attached to the second tag sequence.

86. The method according to claim 85, wherein the ligase comprises a T4 ligase.

87. The method according to any one of claims 83-86, wherein the barcode in the second tag sequence characterizes spatial location information of the second region.

88. The method according to any one of claims 1-87, wherein the second tag sequence comprises a plurality of barcodes.

89. The method according to claim 88, wherein the plurality of barcodes in the second tag sequence are different.

90. The method according to any one of claims 83-89, wherein the nucleic acid sequence in the second binding domain of the adapter in the second tag sequence is located at 3' end of the barcode in the second tag sequence.

91. The method according to any one of claims 1-90, wherein the second tag sequence comprises a unique molecular identification region.

92. The method according to any one of claims 1-91, wherein the second tag sequence comprises a sequencing primer.

93. The method according to any one of claims 1-92, comprising, performing *in situ* hybridization between at least one target molecule in the sample and at least one probe.

94. The method according to any one of claims 1-93, comprising, after the *in situ* hybridization, digesting the sample to obtain the probe attached to the first tag sequence and the probe attached to the second tag sequence.

95. The method according to any one of claims 1-94, comprising amplifying the probe attached to the first tag sequence and the probe attached to the second tag sequence obtained.

96. The method according to any one of claims 1-95, further comprising, after amplifying the probe attached to the first tag sequence and the probe attached to the second tag sequence obtained, sequencing the probe attached to the first tag sequence and the probe attached to the second tag sequence, to determine the composition of the probe attached to the first tag sequence and the probe attached to the second tag sequence.

97. The method according to any one of claims 1-22, wherein, when the target molecule comprises protein, the target molecule binding domain in the probe comprises a protein binding molecule specifically binding to the protein.

98. The method according to claim 97, wherein the protein binding molecule is selected from a group consisting of an antibody, peptide, and peptoid.

99. The method according to any one of claims 1-22, wherein, when the target molecule comprises lipid, the target molecule binding domain in the probe recognizes and binds the target molecule.

100. The method according to any one of claims 1-22, wherein the target molecule comprises DNA derived from the sample.

101. The method according to claim 100, wherein the DNA comprises genomic DNA, open chromatin DNA, a DNA region bound by protein, and/or an exogenous nucleic acid linked with protein, lipid and/or a small molecule compound, the protein, lipid and/or small molecule compound being capable of binding the target molecule within the cell.

102. The method according to any one of claims 100-101, comprising fragmentating the DNA before contacting at least one target molecule in the sample with at least one probe.

103. The method according to claim 102, wherein the fragmentation comprises integrating a sequence containing the probe into the DNA using a transposase-nucleic acid complex and releasing the transposase.

104. The method according to claim 103, wherein the transposase-nucleic acid complex comprises a transposase and a transposon terminal nucleic acid molecule, wherein the transposon terminal nucleic acid molecule comprises the oligonucleotide adapter sequence.

105. The method according to claim 104, wherein the transposase comprises Tn5.

106. The method according to any one of claims 100-105, wherein the DNA comprises a DNA region bound by protein, and the transposase-nucleic acid complex further comprises a portion that directly or indirectly recognizes the protein.

107. The method according to claim 106, wherein the portion that directly or indirectly recognizes the protein comprises one or more of a group consisting of an antibody that specifically binds the protein, and protein A or protein G.

108. The method according to any one of claims 97-107, wherein the reaction blocking modification on the probe in the first region is selected from a group consisting of releasable base modified groups, cleavable linkers, and photosensitive groups.

109. The method according to claim 108, the reaction blocking modification on the probe in the first region is a cleavable linker.

110. The method according to claim 109, wherein the linker is located at 5' end of the probe in the first region.

111. The method according to any one of claims 109-110, wherein the linker is cleavable upon the first processing.

112. The method according to claim 111, wherein the first processing is selected from a group consisting of electron beam processing, acoustic wave, and illumination.

113. The method according to claim 112, wherein the first processing is illumination.

114. The method according to any one of claims 112-113, wherein the light is provided by a light source selected from a group consisting of arc lamp, laser, UV light source, and light emitting diode.

115. The method according to any one of claims 112-114, wherein the light is provided by a UV light source.

116. The method according to any one of claims 108-115, wherein the reaction blocking modification on the probe in the first region is a releasable base modified group.

117. The method according to claim 116, wherein the reaction blocking modification on the probe in the first region comprises at least one releasable base modified group.

118. The method according to any one of claims 116-117, wherein the reaction blocking modification on the probe in the first region comprises at least one releasable photosensitive group.

119. The method according to any one of claims 116-118, wherein the base modified group comprises an NPOM protecting group.

120. The method according to any one of claims 116-119, wherein the base modified group is releasable upon the first processing.

121. The method according to claim 120, wherein the first processing is selected from a group consisting of electron beam processing, acoustic wave, and illumination.

122. The method according to claim 121, wherein the first processing is illumination.

123. The method according to any one of claims 121-122, wherein the light is provided by a light source selected from a group consisting of arc lamp, laser, UV light source, and light emitting diode.

124. The method according to any one of claims 121-123, wherein the light is provided by a UV light source.

125. The method according to any one of claims 97-124, wherein the reaction blocking modification on the probe in the second region is selected from a group consisting of releasable base modified groups, cleavable linkers, and photosensitive groups.

126. The method according to claim 125, wherein the reaction blocking modification on the probe in the second region is a cleavable linker.

127. The method according to claim 126, wherein the linker is located at 5' end of the probe in the second region.

128. The method according to any one of claims 126-127, wherein the linker is cleavable upon the second processing.

129. The method according to claim 128, wherein the second processing is selected from a group consisting of electron beam processing, acoustic wave, and illumination.

130. The method according to claim 129, wherein the second processing is illumination.

131. The method according to any one of claims 129-130, wherein the light is provided by a light source selected from a group consisting of arc lamp, laser, UV light source, and light emitting diode.

132. The method according to any one of claims 129-131, wherein the light is provided by a UV light source.

133. The method according to claim 125, wherein the reaction blocking modification on the probe in the second region is a releasable base modified group.

134. The method according to claim 133, wherein the reaction blocking modification on the probe in the second region comprises at least one releasable base modified group.

135. The method according to any one of claims 133-134, wherein the base modified group comprises an NPOM protecting group.

136. The method according to any one of claims 133-135, wherein the base modified group is releasable upon the second processing.

137. The method according to claim 136, wherein the second processing is selected from a group consisting of electron beam processing, acoustic wave, and illumination.

138. The method according to claim 137, wherein the second processing is illumination.

139. The method according to any one of claims 137-138, wherein the light is provided by a light source selected from a group consisting of arc lamp, laser, UV light source, and light emitting diode.

140. The method according to any one of claims 137-139, wherein the light is provided by a UV light source.

141. The method according to any one of claims 1-22 and 97-140, wherein the first processing and the second processing are the same or different.

142. The method according to any one of claims 1-22 and 97-141, comprising, during or after carrying out the first processing on the first region of the sample, carrying out the second processing on the second region of the sample.

143. The method according to any one of claims 1-22 and 97-142, comprising, after the first processing, administering the first tag molecule containing the first tag sequence to the first region of the sample.

144. The method according to any one of claims 1-22 and 97-143, comprising, after the second processing, administering the second tag molecule containing the second tag sequence to the second region of the sample.

145. The method according to any one of claims 1-22 and 97-144, wherein the probe comprises a first binding domain of an adapter, and the first binding domain of the adapter comprises at least one reaction blocking modification.

146. The method according to claim 145, in the probe, the first binding domain of the adapter is located at 5' end of the target molecule binding domain.

147. The method according to any one of claims 145-146, wherein the probe further comprises a barcode.

148. The method according to claim 147, wherein the barcode characterizes identity of an antibody.

149. The method according to any one of claims 1-22 and 97-148, comprising, upon the first processing, the probe in the first region forming a complex with the first tag sequence and an oligonucleotide adapter, and the oligonucleotide adapter comprises a probe binding domain and a tag binding domain.

150. The method according to any one of claims 1-22 and 97-149, wherein the first tag sequence comprises at least one barcode and a second binding domain of an adapter.

151. The method according to claim 150, comprising, upon the first processing, a nucleic acid sequence in the probe binding domain being complementary to a nucleic acid sequence in the first binding domain of the adapter in the probe in the first region, and a nucleic acid sequence in the tag binding domain being complementary to a nucleic acid sequence in the second binding domain of the adapter in the first tag sequence, thus forming a partially double-stranded structure.

152. The method according to any one of claims 150-151, wherein the second binding domain of the adapter in the first tag sequence is linked to the first binding domain of the adapter in the probe in the first region by a ligase, thus producing the probe attached to the first tag sequence.

153. The method according to claim 152, wherein the ligase comprises a T4 ligase.

154. The method according to any one of claims 150-153, wherein the barcode of the first tag sequence characterizes spatial location information of the first region.

155. The method according to any one of claims 150-154, wherein the first tag sequence comprises a plurality of barcodes.

156. The method according to claim 155, wherein the plurality of barcodes in the first tag sequence are different.

157. The method according to any one of claims 150-156, wherein the nucleic acid sequence in the second binding domain of the adapter in the first tag sequence is located at 3' end of the barcode in the first tag sequence.

158. The method according to any one of claims 1-22 and 97-157, wherein the first tag sequence comprises a unique molecular identification region.

159. The method according to any one of claims 1-22 and 97-158, wherein the first tag sequence comprises a sequencing primer.

160. The method according to any one of claims 1-22 and 97-159, wherein the probe in the second region comprises a first binding domain of an adapter, and the first binding domain of the adapter comprises at least one reaction blocking modification.

161. The method according to claim 160, in the probe, the first binding domain of the adapter is located at 5' end of the target molecule binding domain.

162. The method according to any one of claims 1-22 and 97-161, comprising, upon the second processing, the probe in the second region forming a complex with the second tag sequence and the oligonucleotide adapter.

163. The method according to any one of claims 1-22 and 97-162, wherein the second tag sequence comprises at least one barcode and a second binding domain of an adapter.

164. The method according to claim 163, comprising, upon the second processing, a nucleic acid sequence in the probe binding domain being complementary to a nucleic acid sequence in the first binding domain of the adapter in the probe in the second region, and a nucleic acid sequence in the tag binding domain being complementary to a nucleic acid sequence in the second binding domain of the adapter in the second tag sequence, thus forming a partially double-stranded structure.

165. The method according to any one of claims 163-164, wherein the second binding domain of the adapter in the second tag sequence is linked to the first binding domain of the adapter in the probe in the second region by a ligase, thus producing the probe attached to the second tag sequence.

166. The method according to claim 165, wherein the ligase comprises a T4 ligase.

167. The method according to any one of claims 163-166, wherein the barcode in the second tag sequence characterizes spatial location information of the second region.

168. The method according to any one of claims 163-167, wherein the second tag sequence comprises a plurality of barcodes.

169. The method according to claim 168, wherein the plurality of barcodes in the second tag sequence are different.

170. The method according to any one of claims 163-169, wherein the nucleic acid sequence in the second binding domain of the adapter in the second tag sequence is located at 3' end of the barcode in the second tag sequence.

171. The method according to any one of claims 1-22 and 97-170, wherein the second tag sequence comprises a unique molecular identification region.

172. The method according to any one of claims 1-22 and 97-171, wherein the second tag sequence comprises a sequencing primer.

173. The method according to any one of claims 1-22 and 97-172, wherein the at least one probe specifically recognizes at least one target molecule in the sample.

174. The method according to any one of claims 1-22 and 97-173, comprising digesting the sample to obtain the probe attached to the first tag sequence and the probe attached to the second tag sequence.

175. The method according to any one of claims 1-22 and 97-174, comprising amplifying the probe attached to the first tag sequence and the probe attached to the second tag sequence obtained.

176. The method according to any one of claims 1-22 and 97-175, further comprising, after amplifying the probe attached to the first tag sequence and the probe attached to the second tag sequence obtained, sequencing the probe attached to the first tag sequence and the probe attached to the second tag sequence, to determine the composition of the probe attached to the first tag sequence and the probe attached to the second tag sequence.

177. A combination, comprising: (1) a plurality of probes, each of the probes comprising a target molecule binding domain, a first binding domain of an adapter and at least one reaction blocking modification; (2) an oligonucleotide adapter, the oligonucleotide adapter comprising a probe binding domain and a tag binding domain; and (3) a plurality of tag sequences, each of the tag sequences comprising at least one barcode and a second binding domain of an adapter, wherein a nucleic acid sequence in the probe binding domain is complementary to a nucleic acid sequence in the first binding domain of the adapter, and a nucleic acid sequence in the tag binding domain is complementary to a nucleic acid sequence in the second binding domain of the adapter.

178. The combination according to claim 177, wherein the barcodes in two or more of the plurality of tag sequences are different.

179. The combination according to any one of claims 177-178, wherein the reaction blocking modification is selected from a group consisting of releasable base modified groups, cleavable linkers, and photosensitive groups.

180. The combination according to any one of claims 177-179, wherein the reaction blocking modification is a cleavable linker.

181. The combination according to any one of claims 177-180, wherein the reaction blocking modification is a releasable base modified group.

182. The combination according to any one of claims 177-181, wherein the 1), 2), and 3) exist independently of each other.

183. The combination according to any one of claims 177-182, wherein the 1), 2), and 3) exist as a mixture.

184. A kit, comprising the combination according to any one of claims 177-183.

185. The kit according to claim 184, comprising a transposase.

186. The kit according to any one of claims 184-185, further comprising at least one of a nucleic acid amplifying agent, a reverse transcriptase, a fixing agent, a permeating agent, a ligating agent, and a lysing agent.

187. The kit according to any one of claims 184-186, comprising an instruction for use.

188. The kit according to claim 187, wherein the instruction for use describes the following method: (a) under the condition of specifically binding a probe to a target molecule, contacting at least one target molecule in a sample with at least one probe, the probe containing a target molecule binding domain capable of specifically binding to the target molecule and at least one reaction blocking modification; (b) carrying out a first processing on a first region of the sample, the first processing being capable of at least partially removing the reaction blocking modification on the probe in the first region, to cause the probe in the first region to be attached to a first tag sequence in the tag sequence; (c) carrying out a second processing on a second region that is different from the first region in the sample, the second processing being capable of at least partially removing the reaction blocking modification on the probe in the second region, to cause the probe in the second region to be attached to a second tag sequence in the tag sequence, the second tag sequence being different from the first tag sequence; (d) determining the composition of the probe attached to the first tag sequence and the probe attached to the second tag sequence, and determining the presence and/or content of the target molecule of the first region and the presence and/or content of the target molecule of the second region in the sample from the composition of the probe.
